# EUROPÄISCHE PATENTSCHRIFT

(11) **EP 0 723 962 B1**
(45) Veröffentlichungstag und Bekanntmachung des Hinweises auf die Patenterteilung: **04.07.2001**
(21) Anmeldenummer: 96100468.6
(22) Anmeldetag: 15.01.1996
(51) Int. Cl.: C07D 285/16, A61K 31/54

(54) **Arylalkyl-thiadiazinone**
Arylalkyl-thiadiazinones
Arylalkyl-thiadiazinones

(30) Priorität: 28.01.1995 DE 19502699
(43) Veröffentlichungstag der Anmeldung: 31.07.1996
(73) Patentinhaber: MERCK PATENT GmbH, 64293 Darmstadt (DE)
(72) Erfinder: Jonas, Rochus, Dr., D-64291 Darmstadt (DE); Wolf, Michael, Dr., D-64297 Darmstadt (DE); Klockow, Michael, Dr., D-64380 Rossdorf (DE)

(56) Entgegenhaltungen:
- EP-A- 0 294 647
- EP-A- 0 351 213
- EP-A- 0 539 806
- EP-A- 0 618 201
- DE-A- 3 719 031
- DE-A- 4 134 893

## Beschreibung

Die Erfindung betrifft Arylalkyl-thiadiazinonderivate der Formel I 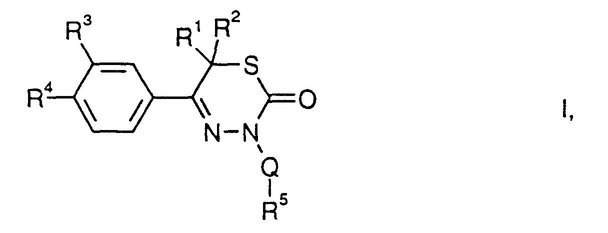 worin
- R¹ und R²: jeweils unabängig voneinander H oder A,
- R³ und R⁴: jeweils unabhängig voneinander -OH, -OR¹⁰, -S-R¹⁰, -SO-R¹⁰, -SO₂-R¹⁰, Hal, Methylendioxy, -NO₂,-NH₂,-NHR¹⁰ oder -NR¹⁰R¹¹,
- R⁵: einen unsubstituierten oder ein- oder zweifach durch R⁶ und/oder R⁷ substituierten Phenylrest,
- Q: Alkylen mit 1-6 C-Atomen,
- R⁶ und R⁷: jeweils unabhängig voneinander -NH₂, -NR⁸R⁹, -NHR¹⁰, -NR¹⁰R¹¹, -NO₂, Hal, -CN, -OA, -COOH oder -COOA,
- R⁸ und R⁹: jeweils unabhängig voneinander H, Acyl mit 1-8 C-Atomen, das durch 1-5 F- und/oder Cl-Atome substituiert sein kann, -COOA, -S-A, -SO-A, -SO₂A,-CONH₂, -CONHA, -CONA₂, -CO-COOH, -CO-COOA, -CO-CONH₂, -CO-CONHA oder -CO-CONA₂,
- A: Alkyl mit 1-6 C-Atomen, das durch 1-5 F- und/oder Cl-Atome substituiert sein kann,
- R¹⁰ und R¹¹: jeweils unabhängig voneinander A, Cycloalkyl mit 3-7 C-Atomen, Methylencycloalkyl mit 4-8 C-Atomen oder Alkenyl mit 2-8 C-Atomen
und
- Hal: F, Cl, Br oder I
bedeuten,
sowie deren physiologisch unbedenklichen Salze.

Thiadiazinone sind beispielsweise aus DE 37 19 031 A1 und DE 41 34 893 bekannt.

Andere Thiadiazinone sind in EP 294647 beschrieben, solche mit cyclischen Aminogruppen kennt man aus EP 618201. Thiadiazinone ohne -Q-R⁵-Rest sind in EP 539806 beschrieben und 2-Indolonderivate sind in EP 351213 offenbart.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I wertvolle pharmakologische Eigenschaften besitzen und zudem eine gute Verträglichkeit aufweisen.

Insbesondere zeigen sie eine Phosphodiesterase IV-Hemmung und können zur Behandlung von asthmatischen Erkrankungen eingesetzt werden. Die antiasthmatische Wirkung kann z.B. nach der Methode von T. Olsson, Acta allergologica 26, 438-447 (1971), bestimmt werden.

Die Verbindungen zeigen außerdem eine hemmende Wirkung auf die Bildung von TNF (Tumor Nekrose Faktor) und eignen sich daher zur Behandlung von allergischen und entzündlichen Krankheiten, Autoimmunkrankheiten und Transplantatabstoßungsreaktionen. Sie können zur Behandlung von Gedächtnisstörungen eingesetzt werden.

Die Verbindungen können daher als Arzneimittelwirkstoffe in der Humanund Veterinärmedizin verwendet werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zu ihrer Herstellung, dadurch gekennzeichnet, daß man eine Verbindung der Formel II, 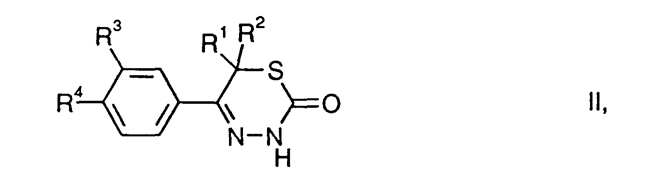 worin R¹, R², R³ und R⁴ die angegebenen Bedeutungen haben mit einer Verbindung der Formel III

R⁵-Q-X III,

worin
- R⁵ und Q: die angegebenen Bedeutungen haben und
- X: Cl, Br, OH oder eine reaktionsfähige veresterte OH-Gruppe bedeutet,
umsetzt,
oder daß man in einer Verbindung der Formel I einen Rest R⁵ in einen anderen Rest R⁵ umwandelt, indem man eine Nitrogruppe reduziert, eine primäre oder eine sekundäre Aminogruppe alkyliert oder acyliert oder eine Cyangruppe hydrolysiert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ und X die angegebenen Bedeutungen haben, umsetzt und/ oder eine Base der Formel I durch Behandeln mit einer Säure in eines ihrer Salze umwandelt.

Vor- und nachstehend haben die Reste R¹, R², R³, R⁴, R⁵, Q und X die bei den Formeln I, II und III angegebenen Bedeutungen, wenn nicht ausdrücklich etwas anderes angegeben ist.

In den Formeln ist Alkyl vorzugsweise unverzweigt, hat vorzugsweise 1, 2, 3 oder 4 C-Atome und steht bevorzugt für Methyl, ferner bevorzugt für Ethyl oder Propyl, weiterhin bevorzugt für Isopropyl, Butyl, Isobutyl, sek.-Butyl, tert.-Butyl, aber auch für n-Pentyl oder Isopentyl.

Cycloalkyl hat vorzugsweise 3-7 C-Atome und steht bevorzugt für Cyclopropyl und Cyclobutyl, weiterhin bevorzugt für Cyclopentyl und Cyclohexyl, ferner auch für Cycloheptyl.

Methylencycloalkyl hat vorzugsweise 4-8 C-Atome und steht bevorzugt für Methylencyclopropyl und Methylencyclobutyl, weiterhin bevorzugt für Methylencyclopentyl und Methylencyclohexyl, ferner auch für Methylencycloheptyl.

Alkenyl ist vorzugsweise Vinyl, Propenyl, iso-Propenyl, Butenyl, Isobutenyl, sek.-Butenyl, ferner bevorzugt ist Pentenyl und iso-Pentenyl.

Alkylen ist vorzugsweise unverzweigt und bedeutet bevorzugt Methylen oder Ethylen, ferner bevorzugt Propylen oder Butylen.

Von den Resten R¹ und R² steht einer vorzugsweise für H, während der andere bevorzugt Propyl oder Butyl, besonders bevorzugt aber Ethyl oder Methyl bedeutet. Ferner bedeuten R¹ und R² auch zusammen bevorzugt jeweils Wasserstoff.

Die Reste R³ und R⁴ können gleich oder verschieden sein und stehen vorzugsweise in der 3- oder 4-Position des Phenylrings. Sie bedeuten beispielsweise unabhängig voneinander Hydroxy, -S-CH₃, -SO-CH₃, -SO₂CH₃, F, Cl, Br oder I oder zusammen Methylendioxy. Besonders bevorzugt stehen sie aber jeweils für Methoxy, Ethoxy, Propoxy oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

Der Rest R⁵ bedeutet vorzugsweise Phenyl. Der Phenylrest ist vorzugsweise ein- oder zweifach substituiert. Bevorzugte Substituenten sind Cyan, Nitro, Amino, Acetamido, Trifluoracetamido, Methoxy und/oder Chlor, ferner sind bevorzugt Methylsulfonamido, Propionylamino, 2-Methylpropionylamino, Isobutyrylamino und/oder Pivalylamino, weiter bevorzugt sind Methoxycarbonylamino, Methoxalylamino, Ureido und/oder Carboxy.

Q-R⁵ ist vorzugsweise Benzyl, 2-, 3- oder 4-Nitrobenzyl, 2-, 3- oder 4-Cyanbenzyl, 2-, 3- oder 4-Aminobenzyl, 2-, 3- oder 4-Acetamidobenzyl, 2-, 3- oder 4-Trifluoracetamidobenzyl, 2-, 3- oder 4-Methoxybenzyl, 2-, 3-oder 4-Chlorbenzyl, weiter bevorzugt ist 2-, 3- oder 4-Methylsulfonamidobenzyl, 2-, 3- oder 4-Propionylaminobenzyl, 2-, 3- oder 4-(2-Methylpropionylamino)-benzyl, 2-, 3- oder 4-Isobutyrylaminobenzyl, 2-, 3- oder 4-Pivalylaminobenzyl, 2-, 3- oder 4-Methoxycarbonylaminobenzyl, 2-, 3-oder 4-Ureidobenzyl, 2-, 3- oder 4-Carboxybenzyl, 2-, 3- oder 4-Methoxalylaminobenzyl, ferner bevorzugt ist 2,3-, 2,4-, 2,5-, 2,6-, 3,4- oder 3,5-Dinitrobenzyl, 2,3-, 2,4-, 2,5-, 2,6-, 3,4- oder 3,5-Diaminobenzyl, 2,3-, 2,4-, 2,5-, 2,6-, 3,4- oder 3,5-Diacetamidobenzyl, 2,3-, 2,4-, 2,5-, 2,6-, 3,4- oder 3,5-Bis-(trifluoracetamido)-benzyl, 2,3-, 2,4-, 2,5-, 2,6-, 3,4- oder 3,5-Dimethoxybenzyl, 2,3-, 2,4-, 2,5-, 2,6-, 3,4- oder 3,5-Dichlorbenzyl, 2,3-, 2,4-, 2,5-, 2,6-, 3,4- oder 3,5-Dimethylsulfonamidobenzyl, 2,3-, 2,4-, 2,5-, 2,6-, 3,4- oder 3,5-Dipropionylaminobenzyl, 2,3-, 2,4-, 2,5-, 2,6-, 3,4-oder 3,5-Bis-(2-Methylpropionylamino)-benzyl, 2,3-, 2,4-, 2,5-, 2,6-, 3,4-oder 3,5-Diisobutyrylaminobenzyl, 2,3-, 2,4-, 2,5-, 2,6-, 3,4- oder 3,5-Di-pivalylaminobenzyl, 2,3-, 2,4-, 2,5-, 2,6-, 3,4- oder 3,5-Dimethoxycarbonylaminobenzyl, 2,3-, 2,4-, 2,5-, 2,6-, 3,4- oder 3,5-Dimethoxalylaminobenzyl, 2,3-, 2,4-, 2,5-, 2,6-, 3,4- oder 3,5-Diureidobenzyl, 2,3-, 2,4-, 2,5-, 2,6-, 3,4- oder 3,5-Dicarboxybenzyl.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch

| | | |
|---|---|---|
| in Ia | R¹ | H, |
| | R² | H oder A, |
| | R³ | OA |

bedeuten;

| | | |
|---|---|---|
| in Ib | R¹ | H, |
| | R² | Methyl oder Ethyl, |
| | R³ und R⁴ | jeweils OA |

bedeuten;

| | | |
|---|---|---|
| in Ic | R¹ | H, |
| | R² | Methyl oder Ethyl, |
| | R³ | OA, |
| | R⁴ | mono-, di- oder trifluorsubstituiertes Alkyl mit 1 bis 6 C-Atomen |

bedeuten;

| | | |
|---|---|---|
| in Id | R¹ | H, |
| | R² | Methyl oder Ethyl, |
| | R³ und R⁴ | OR¹⁰ |
| | R⁵ | einen ein- oder zweifach substituierten Phenylrest |

bedeuten;

| | | |
|---|---|---|
| in le | R¹ und R² | H, |
| | R³ und R⁴ | OA und |
| | R⁵ | einen ein- oder zweifach substituierten Phenylrest bedeuten. |

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formel II haben R¹, R², R³ und R⁴ die angegebenen Bedeutungen, insbesondere die angegebenen bevorzugten Bedeutungen.

In den Verbindungen der Formel III steht Q vorzugsweise für Methylen oder Ethylen, ferner bevorzugt für Propylen oder Butylen.

R⁵ hat in den Verbindungen der Formel III die angegebenen Bedeutungen, insbesondere die angegebenen bevorzugten Bedeutungen, während X Cl, Br, OH oder eine reaktionsfähige veresterte OH-Gruppe bedeutet.

Falls X eine reaktionsfähige veresterte OH-Gruppe bedeutet, so ist diese vorzugsweise Alkylsulfonyloxy mit 1-6 C-Atomen, z.B. Methansulfonyloxy oder Arylsulfonyloxy mit 6-10 C-Atomen, z.B. Benzol-, p-Toluol- oder 1-oder 2-Naphthalinsulfonyloxy.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen.

Die Ausgangsstoffe der Formeln II und III sind teilweise bekannt. Sofern sie nicht bekannt sind, können sie nach an sich bekannten Methoden hergestellt werden.

Thiadiazinone der Formel II und ihre Herstellung sind z.B. in der deutschen Patentanmeldung P 41 34 893 beschrieben.

Die Verbindungen der Formel III werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Im einzelnen erfolgt die Umsetzung der Thiadiazinone der Formel II mit den Verbindungen der Formel III in Gegenwart oder Abwesenheit eines inerten Lösungsmittels bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole und Glykolether wie Ethylenglykol, Diethylenglykol, 2-Methoxyethanol; Nitrile wie Acetonitril; Ether wie Tetrahydrofuran (THF) oder Dioxan; Amide wie Dimethylformamid (DMF); Sulfoxide wie Dimethylsulfoxid. Auch Gemische dieser Lösungsmittel sind geeignet.

Weiterhin kann man in einer Verbindung der Formel I einen Rest R⁵ in einen anderen Rest R⁵ umwandeln, indem man eine Nitrogruppe reduziert, eine primäre oder eine sekundäre Aminogruppe alkyliert oder acyliert oder eine Cyangruppe hydrolysiert.

Ebenso ist es möglich, eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ sowie X die angegebenen Bedeutungen haben, umzusetzen. Die Veretherung der OH-Gruppen erfolgt nach an sich bekannten Methoden, wie sie in Standardwerken der chemischen Literatur (z.B. in Houben-Weyl, Methoden der Organischen Chemie, Georg Thieme Verlag, Stuttgart oder in Organic Reactions, John Wiley & Sons Inc., New York) beschrieben sind, und zwar unter Reaktionsbedingungen, wie sie für die genannten Umsetzungen bekannt und geeignet sind. Dabei kann auch von an sich bekannten, hier nicht näher erwähnten Varianten Gebrauch gemacht werden.

Eine erhaltene Base der Formel I kann mit einer Säure in das zugehörige Säureadditionssalz überführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmonound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Verbindungen der Formel I können eine oder mehrere Asymmetriezentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch-aktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Diese Formel I umschließt alle Stereoisomeren und deren Gemische, z.B. die Racemate.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem, festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten, insbesondere von asthmatischen Erkrankungen sowie bei der therapeutischen Behandlung des menschlichen oder tierischen Körpers verwendet werden.

Dabei werden die erfindungsgemäßen Substanzen in der Regel in Analogie zu bekannten Antiasthmatika, wie z.B. Atrovent® verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

### Beispiel 1

Eine Lösung von 2,8 g 5-(3,4-Dimethoxyphenyl)-6-ethyl-3,6-dihydro-1,3,4-thiadiazin-2-on ("A") [erhältlich durch Umsetzung von 1-(3,4-Dimethoxyphenyl)-2-brombutan-1-on mit Hydrazinthioameisensäuremethylester] in 150 ml Aceton wird mit 3 g 4-Nitrobenzylchlorid in Gegenwart von 4 g Kaliumcarbonat acht Stunden gekocht. Der unlösliche Rückstand wird abfiltriert und die Lösung eingeengt. Man arbeitet wie üblich auf und erhält 3-(4-Nitrobenzyl)-5-(3,4-dimethoxyphenyl)-6-ethyl-3,6-dihydro-1,3,4-thiadiazin-2-on als farbloses Öl.

Analog erhält man durch Umsetzung von "A"
mit 3-Nitrobenzylchlorid:
   3-(3-Nitrobenzyl)-5-(3,4-dimethoxy-phenyl)-6-ethyl-3,6-dihydro-1,3,4-thiadiazin-2-on;
mit 2-Nitrobenzylchlorid:
   3-(2-Nitrobenzyl)-5-(3,4-dimethoxy-phenyl)-6-ethyl-3,6-dihydro-1,3,4-thiadiazin-2-on;
mit 2,3-Dinitrobenzylchlorid:
   3-(2,3-Dinitrobenzyl)-5-(3,4-dimethoxy-phenyl)-6-ethyl-3,6-dihydro-1,3,4-thiadiazin-2-on;
mit 2,4-Dinitrobenzylchlorid:
   3-(2,4-Dinitrobenzyl)-5-(3,4-dimethoxy-phenyl)-6-ethyl-3,6-dihydro-1,3,4-thiadiazin-2-on;
mit 2-Methoxybenzylchlorid:
   3-(2-Methoxybenzyl)-5-(3,4-dimethoxy-phenyl)-6-ethyl-3,6-dihydro-1,3,4-thiadiazin-2-on;
mit 4-Methoxybenzylchlorid:
   3-(4-Methoxybenzyl)-5-(3,4-dimethoxy-phenyl)-6-ethyl-3,6-dihydro-1,3,4-thiadiazin-2-on, F. 120°;
mit 2-Chlorbenzylchlorid:
   3-(2-Chlorbenzyl)-5-(3,4-dimethoxy-phenyl)-6-ethyl-3,6-dihydro-1,3,4-thiadiazin-2-on, F. 77°;
mit 2,6-Dichlorbenzylchlorid:
   3-(2,6-Dichlorbenzyl)-5-(3,4-dimethoxy-phenyl)-6-ethyl-3,6-dihydro-1,3,4-thiadiazin-2-on, F. 187°;
mit 4-Cyanbenzylchlorid:
   3-(4-Cyanbenzyl)-5-(3,4-dimethoxy-phenyl)-6-ethyl-3,6-dihydro-1,3,4-thiadiazin-2-on;
mit 4-Carboxybenzylchlorid:
   3-(4-Carboxybenzyl)-5-(3,4-dimethoxy-phenyl)-6-ethyl-3,6-dihydro-1,3,4-thiadiazin-2-on, F. 106°.

### Beispiel 2

Analog Beispiel 1 erhält man durch Umsetzung von 5-(3,4-Dimethoxyphenyl)-3,6-dihydro-1,3,4-thiadiazin-2-on ("B") mit 4-Nitrobenzylchlorid das 3-(4-Nitrobenzyl)-5-(3,4-dimethoxyphenyl)-3,6-dihydro-1,3,4-thiadiazin-2-on, F. 155°.

Analog erhält man durch Umsetzung von "B"
mit 3-Nitrobenzylchlorid:
   3-(3-Nitrobenzyl)-5-(3,4-dimethoxy-phenyl)-3,6-dihydro-1,3,4-thiadiazin-2-on;
mit 2,4-Dinitrobenzylchlorid:
   3-(2,4-Dinitrobenzyl)-5-(3,4-dimethoxy-phenyl)-3,6-dihydro-1,3,4-thiadiazin-2-on;
mit 4-Methoxybenzylchlorid:
   3-(4-Methoxybenzyl)-5-(3,4-dimethoxy-phenyl)-3,6-dihydro-1,3,4-thiadiazin-2-on;
mit 2-Chlorbenzylchlorid:
   3-(2-Chlorbenzyl)-5-(3,4-dimethoxy-phenyl)-3,6-dihydro-1,3,4-thiadiazin-2-on;
mit 2,6-Dichlorbenzylchlorid:
   3-(2,6-Dichlorbenzyl)-5-(3,4-dimethoxy-phenyl)-3,6-dihydro-1,3,4-thiadiazin-2-on;
mit 4-Cyanbenzylchlorid:
   3-(4-Cyanbenzyl)-5-(3,4-dimethoxy-phenyl)-3,6-dihydro-1,3,4-thiadiazin-2-on.

### Beispiel 3

Analog Beispiel 1 erhält man durch Umsetzung von 5-(3-Methoxy-4-trifluormethoxyphenyl)-6-ethyl-3,6-dihydro-1,3,4-thiadiazin-2-on mit 4-Nitrobenzylchlorid ("C") das 3-(4-Nitrobenzyl)-5-(3-Methoxy-4-trifluormethoxyphenyl)-6-ethyl-3,6-dihydro-1,3,4-thiadiazin-2-on.

Analog erhält man durch Umsetzung von "C"
mit 5-(3-Methoxy-4-difluormethoxyphenyl)-6-ethyl-3,6-dihydro-1,3,4-thiadiazin-2-on:
   3-(4-Nitrobenzyl)-5-(3-methoxy-4-difluormethoxyphenyl)-6-ethyl-3,6-dihydro-1,3,4-thiadiazin-2-on;
mit 5-(3-Methoxy-4-fluormethoxyphenyl)-6-ethyl-3,6-dihydro-1,3,4-thiadiazin-2-on:
   3-(4-Nitrobenzyl)-5-(3-methoxy-4-fluormethoxyphenyl)-6-ethyl-3,6-dihydro-1,3,4-thiadiazin-2-on;
mit 5-(3-Difluormethoxy-4-methoxyphenyl)-6-ethyl-3,6-dihydro-1,3,4-thiadiazin-2-on:
   3-(4-Nitrobenzyl)-5-(3-difluormethoxy-4-methoxyphenyl)-6-ethyl-3,6-dihydro-1,3,4-thiadiazin-2-on;
mit 5-(3-Trifluormethoxy-4-methoxyphenyl)-6-ethyl-3,6-dihydro-1,3,4-thiadiazin-2-on:
   3-(4-Nitrobenzyl)-5-(3-trifluormethoxy-4-methoxyphenyl)-6-ethyl-3,6-dihydro-1,3,4-thiadiazin-2-on;
mit 5-(3-Fluormethoxy-4-methoxyphenyl)-6-ethyl-3,6-dihydro-1,3,4-thiadiazin-2-on:
   3-(4-Nitrobenzyl)-5-(3-fluormethoxy-4-methoxyphenyl)-6-ethyl-3,6-dihydro-1,3,4-thiadiazin-2-on;
mit 5-(3-Methoxy-4-ethoxyphenyl)-6-ethyl-3,6-dihydro-1,3,4-thiadiazin-2-on:
   3-(4-Nitrobenzyl)-5-(3-methoxy-4-ethoxyphenyl)-6-ethyl-3,6-dihydro-1,3,4-thiadiazin-2-on;
mit 5-(3-Ethoxy-4-methoxyphenyl)-6-ethyl-3,6-dihydro-1,3,4-thiadiazin-2-on:
   3-(4-Nitrobenzyl)-5-(3-ethoxy-4-methoxyphenyl)-6-ethyl-3,6-dihydro-1,3,4-thiadiazin-2-on;
mit 5-(3-Hydroxy-4-methoxyphenyl)-6-ethyl-3,6-dihydro-1,3,4-thiadiazin-2-on:
   3-(4-Nitrobenzyl)-5-(3-hydroxy-4-metboxyphenyl)-6-ethyl-3,6-dihydro-1,3,4-thiadiazin-2-on;
mit 5-(4-Methylsulfonylphenyl)-6-ethyl-3,6-dihydro-1,3,4-thiadiazin-2-on:
   3-(4-Nitrobenzyl)-5-(4-methylsulfonylphenyl)-6-ethyl-3,6-dihydro-1,3,4-thiadiazin-2-on;
mit 5-(3,4-Methylenoxyphenyl)-6-ethyl-3,6-dihydro-1,3,4-thiadiazin-2-on:
   3-(4-Nitrobenzyl)-5-(3,4-methylenoxyphenyl)-6-ethyl-3,6-dihydro-1,3,4-thiadiazin-2-on.

### Beispiel 4

Eine Lösung von 3,9 g 3-(4-Nitrobenzyl)-5-(3,4-dimethoxyphenyl)-6-ethyl-3,6-dihydro-1,3,4-thiadiazin-2-on in 40 ml Tetrahydrofuran wird in Gegenwart von Raney-Nickel hydriert. Der Katalysator wird abfiltriert und die Lösung eingeengt. Man erhält nach Umkristallisation 3-(4-Aminobenzyl)-5-(3,4-dimethoxyphenyl)-6-ethyl-3,6-dihydro-1,3,4-thiadiazin-2-on, F. 105°.

Analog erhält man durch Umsetzung
von 3-(3-Nitrobenzyl)-5-(3,4-dimethoxyphenyl)-6-ethyl-3,6-dihydro-1,3,4-thiadiazin-2-on:
   3-(3-Aminobenzyl)-5-(3,4-dimethoxyphenyl)-6-ethyl-3,6-dihydro-1,3,4-thiadiazin-2-on, F. 112°;
von 3-(2-Nitrobenzyl)-5-(3,4-dimethoxyphenyl)-6-ethyl-3,6-dihydro-1,3,4-thiadiazin-2-on:
   3-(2-Aminobenzyl)-5-(3,4-dimethoxyphenyl)-6-ethyl-3,6-dihydro-1,3,4-thiadiazin-2-on;
von 3-(2,3-Dinitrobenzyl)-5-(3,4-dimethoxyphenyl)-6-ethyl-3,6-dihydro-1,3,4-thiadiazin-2-on:
   3-(2,3-Diam inobenzyl)-5-(3,4-dimethoxyphenyl)-6-ethyl-3,6-dihydro-1,3,4-thiadiazin-2-on;
von 3-(2,4-Dinitrobenzyl)-5-(3,4-dimethoxyphenyl)-6-ethyl-3,6-dihydro-1,3,4-thiadiazin-2-on:
   3-(2,4-Diaminobenzyl)-5-(3,4-dimethoxyphenyl)-6-ethyl-3,6-dihydro-1,3,4-thiadiazin-2-on;
von 3-(4-Nitrobenzyl)-5-(3-ethoxy-4-methoxyphenyl)-6-ethyl-3,6-dihydro-1,3,4-thiadiazin-2-on:
   3-(4-Aminobenzyl)-5-(3-ethoxy-4-methoxyphenyl)-6-ethyl-3,6-dihydro-1,3,4-thiadiazin-2-on;
von 3-(4-Nitrobenzyl)-5-(3-methoxy-4-difluormethoxyphenyl)-6-ethyl-3,6-dihydro-1,3,4-thiadiazin-2-on:
   3-(4-Aminobenzyl)-5-(3-methoxy-4-difluormethoxyphenyl)-6-ethyl-3,6-dihydro-1,3,4-thiadiazin-2-on;
von 3-(4-Nitrobenzyl)-5-(3-methoxy-4-fluormethoxyphenyl)-6-ethyl-3,6-dihydro-1,3,4-thiadiazin-2-on:
   3-(4-Aminobenzyl)-5-(3-methoxy-4-fluormethoxyphenyl)-6-ethyl-3,6-dihydro-1,3,4-thiadiazin-2-on;
von 3-(4-Nitrobenzyl)-5-(3-difluormethoxy-4-methoxyphenyl)-6-ethyl-3,6-dihydro-1,3,4-thiadiazin-2-on:
   3-(4-Am inobenzyl)-5-(3-difluormethoxy-4-methoxyphenyl)-6-ethyl-3,6-dihydro-1,3,4-thiadiazin-2-on;
von 3-(4-Nitrobenzyl)-5-(3-trifluormethoxy-4-methoxyphenyl)-6-ethyl-3,6-dihydro-1,3,4-thiadiazin-2-on:
   3-(4-Aminobenzyl)-5-(3-trifluormethoxy-4-methoxyphenyl)-6-ethyl-3,6-dihydro-1,3,4-thiadiazin-2-on;
von 3-(4-Nitrobenzyl)-5-(3-fluormethoxy-4-methoxyphenyl)-6-ethyl-3,6-dihydro-1,3,4-thiadiazin-2-on:
   3-(4-Aminobenzyl)-5-(3-fluormethoxy-4-methoxyphenyl)-6-ethyl-3,6-dihydro-1,3,4-thiadiazin-2-on;
von 3-(4-Nitrobenzyl)-5-(3-methoxy-4-ethoxyphenyl)-6-ethyl-3,6-dihydro-1,3,4-thiadiazin-2-on:
   3-(4-Aminobenzyl)-5-(3-methoxy-4-ethoxyphenyl)-6-ethyl-3,6-dihydro-1,3,4-thiadiazin-2-on;
von 3-(4-Nitrobenzyl)-5-(3-hydroxy-4-methoxyphenyl)-6-ethyl-3,6-dihydro-1,3,4-thiadiazin-2-on:
   3-(4-Aminobenzyl)-5-(3-hydroxy-4-methoxyphenyl)-6-ethyl-3,6-dihydro-1,3,4-thiadiazin-2-on;
von 3-(4-Nitrobenzyl)-5-(4-methylsulfonylphenyl)-6-ethyl-3,6-dihydro-1,3,4-thiadiazin-2-on:
   3-(4-Aminobenzyl)-5-(4-methylsulfonylphenyl)-6-ethyl-3,6-dihydro-1,3,4-thiadiazin-2-on;
von 3-(4-Nitrobenzyl)-5-(3,4-methylenoxyphenyl)-6-ethyl-3,6-dihydro-1,3,4-thiadiazin-2-on:
   3-(4-Aminobenzyl)-5-(3,4-methylenoxyphenyl)-6-ethyl-3,6-dihydro-1,3,4-thiadiazin-2-on;
von 3-(4-Nitrobenzyl)-5-(3-cyclopentyloxy-4-methoxyphenyl)-6-ethyl-3,6-dihydro-1,3,4-thiadiazin-2-on:
   3-(4-Aminobenzyl)-5-(3-cyclopentyloxy-4-methoxyphenyl)-6-ethyl-3,6-dihydro-1,3,4-thiadiazin-2-on, F. 132°;
von 3-(4-Nitrobenzyl)-5-(3,4-dimethoxyphenyl)-3,6-dihydro-1,3,4-thiadiazin-2-on:
   3-(4-Aminobenzyl)-5-(3,4-dimethoxyphenyl)-3,6-dihydro-1,3,4-thiadiazin-2-on;
von 3-(3-Nitrobenzyl)-5-(3,4-dimethoxyphenyl)-3,6-dihydro-1,3,4-thiadiazin-2-on:
   3-(3-Aminobenzyl)-5-(3,4-dimethoxyphenyl)-3,6-dihydro-1,3,4-thiadiazin-2-on;
von 3-(4-Nitrobenzyl)-5-(3-ethoxy-4-methoxyphenyl)-3,6-dihydro-1,3,4-thiadiazin-2-on:
   3-(4-Aminobenzyl)-5-(3-ethoxy-4-methoxyphenyl)-3,6-dihydro-1,3,4-thiadiazin-2-on, F. 176°;
von 3-(4-Nitrobenzyl)-5-(4-ethoxy-3-methoxyphenyl)-3,6-dihydro-1,3,4-thiadiazin-2-on:
   3-(4-Aminobenzyl)-5-(4-ethoxy-3-methoxyphenyl)-3,6-dihydro-1,3,4-thiadiazin-2-on;
von 3-(4-Nitrophenethyl)-5-(3,4-dimethoxyphenyl)-3,6-dihydro-1,3,4-thiadiazin-2-on:
   3-(4-Aminophenethyl)-5-(3,4-dimethoxyphenyl)-3,6-dihydro-1,3,4-thiadiazin-2-on;
von 3-(4-Nitrobenzyl)-5-(3-methoxy-4-difluormethoxyphenyl)-3,6-dihydro-1,3,4-thiadiazin-2-on:
   3-(4-Aminobenzyl)-5-(3-methoxy-4-difluormethoxyphenyl)-3,6-dihydro-1,3,4-thiadiazin-2-on;
von 3-(4-Nitrobenzyl)-5-(3-methoxy-4-fluormethoxyphenyl)-3,6-dihydro-1,3,4-thiadiazin-2-on:
   3-(4-Aminobenzyl)-5-(3-methoxy-4-fluormethoxyphenyl)-3,6-dihydro-1,3,4-thiadiazin-2-on;
von 3-(4-Nitrobenzyl)-5-(3-methoxy-4-trifluormethoxyphenyl)-3,6-dihydro-1,3,4-thiadiazin-2-on:
   3-(4-Aminobenzyl)-5-(3-methoxy-4-trifluormethoxyphenyl)-3,6-dihydro-1,3,4-thiadiazin-2-on;
von 3-(4-Nitrobenzyl)-5-(3-fluormethoxy-4-methoxyphenyl)-3,6-dihydro-1,3,4-thiadiazin-2-on:
   3-(4-Aminobenzyl)-5-(3-fluormethoxy-4-methoxyphenyl)-3,6-dihydro-1,3,4-thiadiazin-2-on;
von 3-(4-Nitrobenzyl)-5-(3-difluormethoxy-4-methoxyphenyl)-3,6-dihydro-1,3,4-thiadiazin-2-on:
   3-(4-Aminobenzyl)-5-(3-difluormethoxy-4-methoxyphenyl)-3,6-dihydro-1,3,4-thiadiazin-2-on;
von 3-(4-Nitrobenzyl)-5-(3-trifluormethoxy-4-methoxyphenyl)-3,6-dihydro-1,3,4-thiadiazin-2-on:
   3-(4-Aminobenzyl)-5-(3-trifluormethoxy-4-methoxyphenyl)-3,6-dihydro-1,3,4-thiadiazin-2-on.

### Beispiel 5

Zu einer gekühlten Lösung von 1,3 g NaOH in 100 ml Wasser wird unter Rühren 10 g 3-(4-Cyanbenzyl)-5-(3,4-dihydroxyphenyl)-6-ethyl-3,6-dihydro-1,3,4-thiadiazin-2-on gegeben und 10 Stunden nachgerührt.

Man erwärmt vorsichtig und leitet dabei einen Luftstrom durch die Lösung.

Anschließend wird gekühlte Schwefelsäure und Wasser zugegeben. Man arbeitet wie üblich auf und erhält 3-(4-Carboxybenzyl)-5-(3,4-dihydroxyphenyl)-6-ethyl-3,6-dihydro-1,3,4-thiadiazin-2-on, F. 106°.

### Beispiel 6

Eine Lösung von 1,4 g 3-(4-Aminobenzyl)-5-(3,4-dimethoxyphenyl)-6-ethyl-3,6-dihydro-1,3,4-thiadiazin-2-on ("D") in 60 ml Dichlormethan und 1 ml Triethylamin wird unter Eiskühlung und Rühren mit 0,8 ml Trifluoracetylchlorid versetzt und 3 Stunden nachgerührt. Man entfernt das Lösungsmittel und arbeitet wie üblich auf. Man erhält nach Umkristallisation aus Isopropanol/Petrolether 1,9 g 3-(4-Trifluoracetamidobenzyl)-5-(3,4-dimethoxyphenyl)-6-ethyl-3,6-dihydro-1,3,4-thiadiazin-2-on, F. 124°.

Analog erhält man durch Umsetzung von "D"
mit Acetylchlorid:
   3-(4-Acetamidobenzyl)-5-(3,4-dimethoxyphenyl)-6-ethyl-3,6-dihydro-1,3,4-thiadiazin-2-on als Öl, MS (El) M⁺ 427;
mit Methylsulfonylchlorid:
   3-(4-Methylsulfonamidobenzyl)-5-(3,4-dimethoxyphenyl)-6-ethyl-3,6-dihydro-1,3,4-thiadiazin-2-on, amorph, MS (El) M⁺ 463;
mit Propionylchlorid:
   3-(4-Propionylaminobenzyl)-5-(3,4-dimethoxyphenyl)-6-ethyl-3,6-dihydro-1,3,4-thiadiazin-2-on, amorph, MS (El) M⁺ 441;
mit Isobutyrylchlorid:
   3-(4-lsobutyrylaminobenzyl)-5-(3,4-dimethoxyphenyl)-6-ethyl-3,6-dihydro-1,3,4-thiadiazin-2-on, amorph, MS (El) M⁺ 455;
mit Chlorameisensäuremethylester:
   3-(4-Methoxycarbonylaminobenzyl)-5-(3,4-dimethoxyphenyl)-6-ethyl-3,6-dihydro-1,3,4-thiadiazin-2-on, F. 141°;
mit Pivalylchlorid:
   3-(4-Pivalylaminobenzyl)-5-(3,4-dimethoxyphenyl)-6-ethyl-3,6-dihydro-1,3,4-thiadiazin-2-on, F. 155°;
mit Cyclopentancarbonsäurechlorid:
   3-(4-Cyclopentylcarbamoylbenzyl)-5-(3,4-dimethoxyphenyl)-6-ethyl-3,6-dihydro-1,3,4-thiadiazin-2-on, F. 115°;
mit Chlorameisensäureethylester:
   3-(4-Ethoxycarbonylaminobenzyl)-5-(3,4-dimethoxyphenyl)-6-ethyl-3,6-dihydro-1,3,4-thiadiazin-2-on, amorph, MS (El) M⁺ 457;
mit Methoxalylchlorid:
   3-(4-Methoxalylaminobenzyl)-5-(3,4-dimethoxyphenyl)-6-ethyl-3,6-dihydro-1,3,4-thiadiazin-2-on, amorph, MS (El) M⁺ 472;
mit Chlorformamid:
   3-(4-Ureidobenzyl)-5-(3,4-dimethoxyphenyl)-6-ethyl-3,6-dihydro-1,3,4-thiadiazin-2-on, F. 140°;
mit Butyrylchlorid:
   3-(4-Butyrylaminobenzyl)-5-(3,4-dimethoxyphenyl)-6-ethyl-3,6-dihydro-1,3,4-thiadiazin-2-on, F. 77°;
mit Pentanoylchlorid:
   3-(4-Pentanoylaminobenzyl)-5-(3,4-dimethoxyphenyl)-6-ethyl-3,6-dihydro-1,3,4-thiadiazin-2-on;
mit Hexanoylchlorid:
   3-(4-Hexanoylaminobenzyl)-5-(3,4-dimethoxyphenyl)-6-ethyl-3,6-dihydro-1,3,4-thiadiazin-2-on, amorph;
mit Pentafluorpropionylchlorid:
   3-(4-Pentafluorpropionylaminobenzyl)-5-(3,4-dimethoxyphenyl)-6-ethyl-3,6-dihydro-1,3,4-thiadiazin-2-on, F. 113°.

Analog erhält man durch Umsetzung von 3-(4-Aminobenzyl)-5-(3-ethoxy-4-methoxyphenyl)-6-ethyl-3,6-dihydro-1,3,4-thiadiazin-2-on
mit Trifluoracetylchlorid:
   3-(4-Trifluoracetamidobenzyl)-5-(3-ethoxy-4-methoxyphenyl)-6-ethyl-3,6-dihydro-1,3,4-thiadiazin-2-on, F. 155°;
mit Cyclopentancarbonsäurechlorid:
   3-(4-Cyclopentylcarbamoylbenzyl)-5-(3-ethoxy-4-methoxyphenyl)-6-ethyl-3,6-dihydro-1,3,4-thiadiazin-2-on;
mit Acetylchlorid:
   3-(4-Acetamidobenzyl)-5-(3-ethoxy-4-methoxyphenyl)-6-ethyl-3,6-dihydro-1,3,4-thiadiazin-2-on;
mit Methylsulfonylchlorid:
   3-(4-Methylsulfonylamidobenzyl)-5-(3-ethoxy-4-methoxyphenyl)-6-ethyl-3,6-dihydro-1,3,4-thiadiazin-2-on;
mit Propionylchlorid:
   3-(4-Propionylaminobenzyl)-5-(3-ethoxy-4-methoxyphenyl)-6-ethyl-3,6-dihydro-1,3,4-thiadiazin-2-on, F. 136°;
mit Isobutyrylchlorid:
   3-(4-lsobutyrylaminobenzyl)-5-(3-ethoxy-4-methoxyphenyl)-6-ethyl-3,6-dihydro-1,3,4-thiadiazin-2-on;
mit Chlorameisensäuremethylester:
   3-(4-Methoxycarbonylaminobenzyl)-5-(3-ethoxy-4-methoxyphenyl)-6-ethyl-3,6-dihydro-1,3,4-thiadiazin-2-on;
mit Chlorameisensäureethylester:
   3-(4-Ethoxycarbonylaminobenzyl)-5-(3-ethoxy-4-methoxyphenyl)-6-ethyl-3,6-dihydro-1,3,4-thiadiazin-2-on, F. 160°;
mit Methoxalylchlorid:
   3-(4-Methoxalylaminobenzyl)-5-(3-ethoxy-4-methoxyphenyl)-6-ethyl-3,6-dihydro-1,3,4-thiadiazin-2-on;
mit Chlorformamid:
   3-(4-Ureidobenzyl)-5-(3-ethoxy-4-methoxyphenyl)-6-ethyl-3,6-dihydro-1,3,4-thiadiazin-2-on;
mit Butyrylchlorid:
   3-(4-Butyrylaminobenzyl)-5-(3-ethoxy-4-methoxyphenyl)-6-ethyl-3,6-dihydro-1,3,4-thiadiazin-2-on;
mit Pentanoylchlorid:
   3-(4-Pentanoylaminobenzyl)-5-(3-ethoxy-4-methoxyphenyl)-6-ethyl-3,6-dihydro-1,3,4-thiadiazin-2-on;
mit Pivalylchlorid:
   3-(4-Pivalylaminobenzyl)-5-(3-ethoxy-4-methoxyphenyl)-6-ethyl-3,6-dihydro-1,3,4-thiadiazin-2-on;
mit Hexanoylchlorid:
   3-(4-Hexanoylaminobenzyl)-5-(3-ethoxy-4-methoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-ethoxy-4-methoxyphenyl)-6-ethyl-3,6-dihydro-1,3,4-thiadiazin-2-on.

Analog erhält man durch Umsetzung von 3-(4-Aminobenzyl)-5-(3-cyclopentyloxy-4-methoxyphenyl)-6-ethyl-3,6-dihydro-1,3,4-thiadiazin-2-on
mit Trifluoracetylchlorid:
   3-(4-Trifluoracetam idobenzyl)-5-(3-cyclopentyloxy-4-methoxyphenyl)-6-ethyl-3,6-dihydro-1,3,4-thiadiazin-2-on;
mit Acetylchlorid:
   3-(4-Acetam idobenzyl)-5-(3-cyclopentyloxy-4-methoxyphenyl)-6-ethyl-3,6-dihydro-1,3,4-thiadiazin-2-on, amorph, MS (El) M⁺ 481;
mit Methylsulfonylchlorid:
   3-(4-Methylsulfonamidobenzyl)-5-(3-cyclopentyloxy-4-methoxyphenyl)-6-ethyl-3,6-dihydro-1,3,4-thiadiazin-2-on;
mit Propionylchlorid:
   3-(4-Propionylam inobenzyl)-5-(3-cyclopentyloxy-4-methoxyphenyl)-6-ethyl-3,6-dihydro-1,3,4-thiadiazin-2-on;
mit Isobutyrylchlorid:
   3-(4-lsobutyrylaminobenzyl)-5-(3-cyclopentyloxy-4-methoxyphenyl)-6-ethyl-3,6-dihydro-1,3,4-thiadiazin-2-on;
mit Chlorameisensäuremethylester:
   3-(4-Methoxycarbonylaminobenzyl)-5-(3-cyclopentyloxy-4-methoxyphenyl)-6-ethyl-3,6-dihydro-1,3,4-thiadiazin-2-on;
mit Pivalylchlorid:
   3-(4-Pivalylaminobenzyl)-5-(3-cyclopentyloxy-4-methoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-cyclopentyloxy-4-methoxyphenyl)-6-ethyl-3,6-dihydro-1,3,4-thiadiazin-2-on;
mit Chlorameisensäureethylester:
   3-(4-Ethoxycarbonylaminobenzyl)-5-(3-cyclopentyloxy-4-methoxyphenyl)-6-ethyl-3,6-dihydro-1,3,4-thiadiazin-2-on, F. 146°;
mit Methoxalylchlorid:
   3-(4-Methoxalylaminobenzyl)-5-(3-cyclopentyloxy-4-methoxyphenyl)-6-ethyl-3,6-dihydro-1,3,4-thiadiazin-2-on;
mit Chlorformamid:
   3-(4-Ureidobenzyl)-5-(3-cyclopentyloxy-4-methoxyphenyl)-6-ethyl-3,6-dihydro-1,3,4-thiadiazin-2-on;
mit Butyrylchlorid:
   3-(4-Butyrylaminobenzyl)-5-(3-cyclopentyloxy-4-methoxyphenyl)-6-ethyl-3,6-dihydro-1,3,4-thiadiazin-2-on;
mit Pentanoylchlorid:
   3-(4-Pentanoylaminobenzyl)-5-(3-cyclopentyloxy-4-methoxyphenyl)-6-ethyl-3,6-dihydro-1,3,4-thiadiazin-2-on;
mit Hexanoylchlorid:
   3-(4-Hexanoylaminobenzyl)-5-(3-cyclopentyloxy-4-methoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-cyclopentyloxy-4-methoxyphenyl)-6-ethyl-3,6-dihydro-1,3,4-thiadiazin-2-on.

Analog erhält man durch Umsetzung von 3-(4-Aminophenethyl)-5-(3,4-dimethoxyphenyl)-6-ethyl-3,6-dihydro-1,3,4-thiadiazin-2-on
mit Trifluoracetylchlorid:
   3-(4-Trifluoracetamidophenethyl)-5-(3,4-dimethoxyphenyl)-6-ethyl-3,6-dihydro-1,3,4-thiadiazin-2-on;
mit Acetylchlorid:
   3-(4-Acetamidophenethyl)-5-(3,4-dimethoxyphenyl)-6-ethyl-3,6-dihydro-1,3,4-thiadiazin-2-on, F. 112°;
mit Methylsulfonylchlorid:
   3-(4-Methylsulfonamidophenethyl)-5-(3,4-dimethoxyphenyl)-6-ethyl-3,6-dihydro-1,3,4-thiadiazin-2-on;
mit Propionylchlorid:
   3-(4-Propionylaminophenethyl)-5-(3,4-dimethoxyphenyl)-6-ethyl-3,6-dihydro-1,3,4-thiadiazin-2-on;
mit Isobutyrylchlorid:
   3-(4-Isobutyrylaminophenethyl)-5-(3,4-dimethoxyphenyl)-6-ethyl-3,6-dihydro-1,3,4-thiadiazin-2-on;
mit Chlorameisensäuremethylester:
   3-(4-Methoxycarbonylaminophenethyl)-5-(3,4-dimethoxyphenyl)-6-ethyl-3,6-dihydro-1,3,4-thiadiazin-2-on;
mit Chlorameisensäureethylester:
   3-(4-Ethoxycarbonylaminophenethyl)-5-(3,4-dimethoxyphenyl)-6-ethyl-3,6-dihydro-1,3,4-thiadiazin-2-on;
mit Methoxalylchlorid:
   3-(4-Methoxalylaminophenethyl)-5-(3,4-dimethoxyphenyl)-6-ethyl-3,6-dihydro-1,3,4-thiadiazin-2-on;
mit Chlorformamid:
   3-(4-Ureidophenethyl)-5-(3,4-dimethoxyphenyl)-6-ethyl-3,6-dihydro-1,3,4-thiadiazin-2-on;
mit Butyrylchlorid:
   3-(4-Butyrylaminophenethyl)-5-(3,4-dimethoxyphenyl)-6-ethyl-3,6-dihydro-1,3,4-thiadiazin-2-on;
mit Cyclopentancarbonsäurechlorid:
   3-(4-Cyclopentylcarbamoylphenethyl)-5-(3,4-dimethoxyphenyl)-6-ethyl-3,6-dihydro-1,3,4-thiadiazin-2-on;
mit Pentanoylchlorid:
   3-(4-Pentanoylaminophenethyl)-5-(3,4-dimethoxyphenyl)-6-ethyl-3,6-dihydro-1,3,4-thiadiazin-2-on;
mit Hexanoylchlorid:
   3-(4-Hexanoylaminophenethyl)-5-(3,4-dimethoxyphenyl)-6-ethyl-3,6-dihydro-1,3,4-thiadiazin-2-on;
mit Pentafluorpropionylchlorid:
   3-(4-Pentafluorpropionylaminophenethyl)-5-(3,4-dimethoxyphenyl)-6-ethyl-3,6-dihydro-1,3,4-thiadiazin-2-on;
mit Pivalylchlorid:
   3-(4-Pivalylaminophenethyl)-5-(3,4-dimethoxyphenyl)-6-ethyl-3,6-dihydro-1,3,4-thiadiazin-2-on.

Analog erhält man durch Umsetzung von 3-(3-Aminobenzyl)-5-(3,4-dimethoxyphenyl)-6-ethyl-3,6-dihydro-1,3,4-thiadiazin-2-on
mit Trifluoracetylchlorid:
   3-(3-Trifluoracetamidobenzyl)-5-(3,4-dimethoxyphenyl)-6-ethyl-3,6-dihydro-1,3,4-thiadiazin-2-on, amorph, MS (EI) M⁺ 481;
mit Acetylchlorid:
   3-(3-Acetamidobenzyl)-5-(3,4-dimethoxyphenyl)-6-ethyl-3,6-dihydro-1,3,4-thiadiazin-2-on;
mit Methylsulfonylchlorid:
   3-(3-Methylsulfonamidobenzyl)-5-(3,4-dimethoxyphenyl)-6-ethyl-3,6-dihydro-1,3,4-thiadiazin-2-on;
mit Propionylchlorid:
   3-(3-Propionylaminobenzyl)-5-(3,4-dimethoxyphenyl)-6-ethyl-3,6-dihydro-1,3,4-thiadiazin-2-on, F. 159°;
mit Cyclopentancarbonsäurechlorid:
   3-(3-Cyclopentylcarbamoylbenzyl)-5-(3,4-dimethoxyphenyl)-6-ethyl-3,6-dihydro-1,3,4-thiadiazin-2-on;
mit Isobutyrylchlorid:
   3-(3-Isobutyrylam inobenzyl)-5-(3,4-dimethoxyphenyl)-6-ethyl-3,6-dihydro-1,3,4-thiadiazin-2-on;
mit Chlorameisensäuremethylester:
   3-(3-Methoxycarbonylam inobenzyl)-5-(3,4-dimethoxyphenyl)-6-ethyl-3,6-dihydro-1,3,4-thiadiazin-2-on;
mit Chlorameisensäureethylester:
   3-(3-Ethoxycarbonylaminobenzyl)-5-(3,4-dimethoxyphenyl)-6-ethyl-3,6-dihydro-1,3,4-thiadiazin-2-on, amorph;
mit Methoxalylchlorid:
   3-(3-Methoxalylaminobenzyl)-5-(3,4-dimethoxyphenyl)-6-ethyl-3,6-dihydro-1,3,4-thiadiazin-2-on;
mit Chlorformamid:
   3-(3-Ureidobenzyl)-5-(3,4-dimethoxyphenyl)-6-ethyl-3,6-dihydro-1,3,4-thiadiazin-2-on;
mit Butyrylchlorid:
   3-(3-Butyrylaminobenzyl)-5-(3,4-dimethoxyphenyl)-6-ethyl-3,6-dihydro-1,3,4-thiadiazin-2-on;
mit Pentanoylchlorid:
   3-(3-Pentanoylaminobenzyl)-5-(3,4-dimethoxyphenyl)-6-ethyl-3,6-dihydro-1,3,4-thiadiazin-2-on;
mit Hexanoylchlorid:
   3-(3-Hexanoylaminobenzyl)-5-(3,4-dimethoxyphenyl)-6-ethyl-3,6-dihydro-1,3,4-thiadiazin-2-on;
mit Pentafluorpropionylchlorid:
   3-(3-Pentafluorpropionylaminobenzyl)-5-(3,4-dimethoxyphenyl)-6-ethyl-3,6-dihydro-1,3,4-thiadiazin-2-on;
mit Pivalylchlorid:
   3-(3-Pivalylaminobenzyl)-5-(3,4-dimethoxyphenyl)-6-ethyl-3,6-dihydro-1,3,4-thiadiazin-2-on.

Analog erhält man durch Umsetzung von 3-(4-Aminobenzyl)-5-(3-fluormethoxy-4-methoxyphenyl)-6-ethyl-3,6-dihydro-1,3,4-thiadiazin-2-on
mit Trifluoracetylchlorid:
   3-(4-Trifluoracetamidobenzyl)-5-(3-fluormethoxy-4-methoxyphenyl)-6-ethyl-3,6-dihydro-1,3,4-thiadiazin-2-on;
mit Acetylchlorid:
   3-(4-Acetamidobenzyl)-5-(3-fluormethoxy-4-methoxyphenyl)-6-ethyl-3,6-dihydro-1,3,4-thiadiazin-2-on;
mit Methylsulfonylchlorid:
   3-(4-Methylsulfonamidobenzyl)-5-(3-fluormethoxy-4-methoxyphenyl)-6-ethyl-3,6-dihydro-1,3,4-thiadiazin-2-on;
mit Propionylchlorid:
   3-(4-Propionylaminobenzyl)-5-(3-ftuormethoxy-4-methoxyphenyl)-6-ethyl-3,6-dihydro-1,3,4-thiadiazin-2-on;
mit Isobutyrylchlorid:
   3-(4-lsobutyrylaminobenzyl)-5-(3-fluormethoxy-4-methoxyphenyl)-6-ethyl-3,6-dihydro-1,3,4-thiadiazin-2-on;
mit Chlorameisensäuremethylester:
   3-(4-Methoxycarbonylaminobenzyl)-5-(3-fluormethoxy-4-methoxy-phenyl)-6-ethyl-3,6-dihydro-1,3,4-thiadiazin-2-on;
mit Chlorameisensäureethylester:
   3-(4-Ethoxycarbonylaminobenzyl)-5-(3-fluormethoxy-4-methoxy-phenyl)-6-ethyl-3,6-dihydro-1,3,4-thiadiazin-2-on;
mit Methoxalylchlorid:
   3-(4-Methoxalylam inobenzyl)-5-(3-fluormethoxy-4-methoxyphenyl)-6-ethyl-3,6-dihydro-1,3,4-thiadiazin-2-on;
mit Chlorformamid:
   3-(4-Ureidobenzyl)-5-(3-fluormethoxy-4-methoxyphenyl)-6-ethyl-3,6-dihydro-1,3,4-thiadiazin-2-on;
mit Butyrylchlorid:
   3-(4-Butyrylaminobenzyl)-5-(3-fluormethoxy-4-methoxyphenyl)-6-ethyl-3,6-dihydro-1,3,4-thiadiazin-2-on;
mit Pentanoylchlorid:
   3-(4-Pentanoylaminobenzyl)-5-(3-fluormethoxy-4-methoxyphenyl)-6-ethyl-3,6-dihydro-1,3,4-thiadiazin-2-on;
mit Hexanoylchlorid:
   3-(4-Hexanoylaminobenzyl)-5-(3-fluormethoxy-4-methoxyphenyl)-6-ethyl-3,6-dihydro-1,3,4-thiadiazin-2-on;
mit Pentafluorpropionylchlorid:
   3-(4-Pentafluorpropionylam inobenzyl)-5-(3-fluormethoxy-4-methoxy-phenyl)-6-ethyl-3,6-dihydro-1,3,4-thiadiazin-2-on;
mit Pivalylchlorid:
   3-(4-Pivalylaminobenzyl)-5-(3-fluormethoxy-4-methoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-fluormethoxy-4-methoxy-phenyl)-6-ethyl-3,6-dihydro-1,3,4-thiadiazin-2-on.

Analog erhält man durch Umsetzung von 3-(4-Aminobenzyl)-5-(3-difluormethoxy-4-methoxyphenyl)-6-ethyl-3,6-dihydro-1,3,4-thiadiazin-2-on
mit Trifluoracetylchlorid:
   3-(4-Trifluoracetamidobenzyl)-5-(3-difluormethoxy-4-methoxy-phenyl)-6-ethyl-3,6-dihydro-1,3,4-thiadiazin-2-on;
mit Acetylchlorid:
   3-(4-Acetamidobenzyl)-5-(3-difluormethoxy-4-methoxyphenyl)-6-ethyl-3,6-dihydro-1,3,4-thiadiazin-2-on;
mit Methylsulfonylchlorid:
   3-(4-Methylsulfonamidobenzyl)-5-(3-difluormethoxy-4-methoxy-phenyl)-6-ethyl-3,6-dihydro-1,3,4-thiadiazin-2-on;
mit Propionylchlorid:
   3-(4-Propionylaminobenzyl)-5-(3-difluormethoxy-4-methoxyphenyl)-6-ethyl-3,6-dihydro-1,3,4-thiadiazin-2-on;
mit Isobutyrylchlorid:
   3-(4-Isobutyrylaminobenzyl)-5-(3-difluormethoxy-4-methoxyphenyl)-6-ethyl-3,6-dihydro-1,3,4-thiadiazin-2-on;
mit Chlorameisensäuremethylester:
   3-(4-Methoxycarbonylaminobenzyl)-5-(3-difluormethoxy-4-methoxy-phenyl)-6-ethyl-3,6-dihydro-1,3,4-thiadiazin-2-on;
mit Chlorameisensäureethylester:
   3-(4-Ethoxycarbonylaminobenzyl)-5-(3-difluormethoxy-4-methoxy-phenyl)-6-ethyl-3,6-dihydro-1,3,4-thiadiazin-2-on;
mit Methoxalylchlorid:
   3-(4-Methoxalylaminobenzyl)-5-(3-difluormethoxy-4-methoxyphenyl)-6-ethyl-3,6-dihydro-1,3,4-thiadiazin-2-on;
mit Chlorformamid:
   3-(4-Ureidobenzyl)-5-(3-difluormethoxy-4-methoxyphenyl)-6-ethyl-3,6-dihydro-1,3,4-thiadiazin-2-on;
mit Butyrylchlorid:
   3-(4-Butyrylaminobenzyl)-5-(3-difluormethoxy-4-methoxyphenyl)-6-ethyl-3,6-dihydro-1,3,4-thiadiazin-2-on;
mit Pentanoylchlorid:
   3-(4-Pentanoylaminobenzyl)-5-(3-difluormethoxy-4-methoxyphenyl)-6-ethyl-3,6-dihydro-1,3,4-thiadiazin-2-on;
mit Hexanoylchlorid:
   3-(4-Hexanoylaminobenzyl)-5-(3-difluormethoxy-4-methoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-difluormethoxy-4-methoxyphenyl)-6-ethyl-3,6-dihydro-1,3,4-thiadiazin-2-on;
mit Pivalylchlorid:
   3-(4-Pivalylaminobenzyl)-5-(3-difluormethoxy-4-methoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-difluormethoxy-4-methoxyphenyl)-6-ethyl-3,6-dihydro-1,3,4-thiadiazin-2-on.

Analog erhält man durch Umsetzung von 3-(4-Aminobenzyl)-5-(3-trifluormethoxy-4-methoxyphenyl)-6-ethyl-3,6-dihydro-1,3,4-thiadiazin-2-on
mit Trifluoracetylchlorid:
   3-(4-Trifluoracetamidobenzyl)-5-(3-trifluormethoxy-4-methoxyphenyl)-6-ethyl-3,6-dihydro-1,3,4-thiadiazin-2-on;
mit Acetylchlorid:
   3-(4-Acetamidobenzyl)-5-(3-trifluormethoxy-4-methoxyphenyl)-6-ethyl-3,6-dihydro-1,3,4-thiadiazin-2-on;
mit Methylsulfonylchlorid:
   3-(4-Methylsulfonamidobenzyl)-5-(3-trifluormethoxy-4-methoxyphenyl)-6-ethyl-3,6-dihydro-1,3,4-thiadiazin-2-on;
mit Propionylchlorid:
   3-(4-Propionylaminobenzyl)-5-(3-trifluormethoxy-4-methoxyphenyl)-6-ethyl-3,6-dihydro-1,3,4-thiadiazin-2-on;
mit Isobutyrylchlorid:
   3-(4-Isobutyrylaminobenzyl)-5-(3-trifluormethoxy-4-methoxyphenyl)-6-ethyl-3,6-dihydro-1,3,4-thiadiazin-2-on;
mit Chlorameisensäuremethylester:
   3-(4-Methoxycarbonylaminobenzyl)-5-(3-trifluormethoxy-4-methoxy-phenyl)-6-ethyl-3,6-dihydro-1,3,4-thiadiazin-2-on;
mit Chlorameisensäureethylester:
   3-(4-Ethoxycarbonylaminobenzyl)-5-(3-trifluormethoxy-4-methoxyphenyl)-6-ethyl-3,6-dihydro-1,3,4-thiadiazin-2-on;
mit Methoxalylchlorid:
   3-(4-Methoxalylaminobenzyl)-5-(3-trifluormethoxy-4-methoxyphenyl)-6-ethyl-3,6-dihydro-1,3,4-thiadiazin-2-on;
mit Chlorformamid:
   3-(4-Ureidobenzyl)-5-(3-trifluormethoxy-4-methoxyphenyl)-6-ethyl-3,6-dihydro-1,3,4-thiadiazin-2-on;
mit Butyrylchlorid:
   3-(4-Butyrylaminobenzyl)-5-(3-trifluormethoxy-4-methoxyphenyl)-6-ethyl-3,6-dihydro-1,3,4-thiadiazin-2-on;
mit Pentanoylchlorid:
   3-(4-Pentanoylaminobenzyl)-5-(3-trifluormethoxy-4-methoxyphenyl)-6-ethyl-3,6-dihydro-1,3,4-thiadiazin-2-on;
mit Hexanoylchlorid:
   3-(4-Hexanoylaminobenzyl)-5-(3-trifluormethoxy-4-methoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-trifluormethoxy-4-methoxyphenyl)-6-ethyl-3,6-dihydro-1,3,4-thiadiazin-2-on;
mit Pivalylchlorid:
   3-(4-Pivalylaminobenzyl)-5-(3-trifluormethoxy-4-methoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-trifluormethoxy-4-methoxy-phenyl)-6-ethyl-3,6-dihydro-1,3,4-thiadiazin-2-on.

Analog erhält man durch Umsetzung von 3-(4-Aminobenzyl)-5-(3-methoxy-4-fluormethoxyphenyl)-6-ethyl-3,6-dihydro-1,3,4-thiadiazin-2-on
mit Trifluoracetylchlorid:
   3-(4-Trifluoracetamidobenzyl)-5-(3-methoxy-4-fluormethoxyphenyl)-6-ethyl-3,6-dihydro-1,3,4-thiadiazin-2-on;
mit Acetylchlorid:
   3-(4-Acetamidobenzyl)-5-(3-methoxy-4-fluormethoxyphenyl)-6-ethyl-3,6-dihydro-1,3,4-thiadiazin-2-on;
mit Methylsulfonylchlorid:
   3-(4-Methylsulfonamidobenzyl)-5-(3-methoxy-4-fluormethoxyphenyl)-6-ethyl-3,6-dihydro-1,3,4-thiadiazin-2-on;
mit Propionylchlorid:
   3-(4-Propionylaminobenzyl)-5-(3-methoxy-4-fluormethoxyphenyl)-6-ethyl-3,6-dihydro-1,3,4-thiadiazin-2-on;
mit Isobutyrylchlorid:
   3-(4-lsobutyrylaminobenzyl)-5-(3-methoxy-4-fluormethoxyphenyl)-6-ethyl-3,6-dihydro-1,3,4-thiadiazin-2-on;
mit Chlorameisensäuremethylester:
   3-(4-Methoxycarbonylaminobenzyl)-5-(3-methoxy-4-fluormethoxyphenyl)-6-ethyl-3,6-dihydro-1,3,4-thiadiazin-2-on;
mit Chlorameisensäureethylester:
   3-(4-Ethoxycarbonylaminobenzyl)-5-(3-methoxy-4-fluormethoxyphenyl)-6-ethyl-3,6-dihydro-1,3,4-thiadiazin-2-on;
mit Methoxalylchlorid:
   3-(4-Methoxalylaminobenzyl)-5-(3-methoxy-4-fluormethoxyphenyl)-6-ethyl-3,6-dihydro-1,3,4-thiadiazin-2-on;
mit Chlorformamid:
   3-(4-Ureidobenzyl)-5-(3-methoxy-4-fluormethoxyphenyl)-6-ethyl-3,6-dihydro-1,3,4-thiadiazin-2-on;
mit Butyrylchlorid:
   3-(4-Butyrylaminobenzyl)-5-(3-methoxy-4-fluormethoxyphenyl)-6-ethyl-3,6-dihydro-1,3,4-thiadiazin-2-on;
mit Pentanoylchlorid:
   3-(4-Pentanoylaminobenzyl)-5-(3-methoxy-4-fluormethoxyphenyl)-6-ethyl-3,6-dihydro-1,3,4-thiadiazin-2-on;
mit Hexanoylchlorid:
   3-(4-Hexanoylaminobenzyl)-5-(3-methoxy-4-fluormethoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-methoxy-4-fluormethoxy-phenyl)-6-ethyl-3,6-dihydro-1,3,4-thiadiazin-2-on;
mit Pivalylchlorid:
   3-(4-Pivalylaminobenzyl)-5-(3-methoxy-4-fluormethoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-methoxy-4-fluormethoxy-phenyl)-6-ethyl-3,6-dihydro-1,3,4-thiadiazin-2-on.

Analog erhält man durch Umsetzung von 3-(4-Aminobenzyl)-5-(3-methoxy-4-difluormethoxyphenyl)-6-ethyl-3,6-dihydro-1,3,4-thiadiazin-2-on
mit Trifluoracetylchlorid:
   3-(4-Trifluoracetamidobenzyl)-5-(3-methoxy-4-difluormethoxy-phenyl)-6-ethyl-3,6-dihydro-1,3,4-thiadiazin-2-on;
mit Acetylchlorid:
   3-(4-Acetamidobenzyl)-5-(3-methoxy-4-difluormethoxyphenyl)-6-ethyl-3,6-dihydro-1,3,4-thiadiazin-2-on;
mit Methylsulfonylchlorid:
   3-(4-Methylsulfonamidobenzyl)-5-(3-methoxy-4-difluormethoxy-phenyl)-6-ethyl-3,6-dihydro-1,3,4-thiadiazin-2-on;
mit Propionylchlorid:
   3-(4-Propionylaminobenzyl)-5-(3-methoxy-4-difluormethoxyphenyl)-6-ethyl-3,6-dihydro-1,3,4-thiadiazin-2-on;
mit Isobutyrylchlorid:
   3-(4-lsobutyrylaminobenzyl)-5-(3-methoxy-4-difluormethoxyphenyl)-6-ethyl-3,6-dihydro-1,3,4-thiadiazin-2-on;
mit Chlorameisensäuremethylester:
   3-(4-Methoxycarbonylaminobenzyl)-5-(3-methoxy-4-difluormethoxyphenyl)-6-ethyl-3,6-dihydro-1,3,4-thiadiazin-2-on;
mit Chlorameisensäureethylester:
   3-(4-Ethoxycarbonylaminobenzyl)-5-(3-methoxy-4-difluormethoxyphenyl)-6-ethyl-3,6-dihydro-1,3,4-thiadiazin-2-on;
mit Methoxalylchlorid:
   3-(4-Methoxalylaminobenzyl)-5-(3-methoxy-4-difluormethoxyphenyl)-6-ethyl-3,6-dihydro-1,3,4-thiadiazin-2-on;
mit Chlorformamid:
   3-(4-Ureidobenzyl)-5-(3-methoxy-4-difluormethoxyphenyl)-6-ethyl-3,6-dihydro-1,3,4-thiadiazin-2-on;
mit Butyrylchlorid:
   3-(4-Butyrylaminobenzyl)-5-(3-methoxy-4-difluormethoxyphenyl)-6-ethyl-3,6-dihydro-1,3,4-thiadiazin-2-on;
mit Pentanoylchlorid:
   3-(4-Pentanoylaminobenzyl)-5-(3-methoxy-4-difluormethoxyphenyl)-6-ethyl-3,6-dihydro-1,3,4-thiadiazin-2-on;
mit Hexanoylchlorid:
   3-(4-Hexanoylaminobenzyl)-5-(3-methoxy-4-difluormethoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-methoxy-4-difluormethoxyphenyl)-6-ethyl-3,6-dihydro-1,3,4-thiadiazin-2-on;
mit Pivalylchlorid:
   3-(4-Pivalylaminobenzyl)-5-(3-methoxy-4-difluormethoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-methoxy-4-difluormethoxyphenyl)-6-ethyl-3,6-dihydro-1,3,4-thiadiazin-2-on.

Analog erhält man durch Umsetzung von 3-(4-Aminobenzyl)-5-(3-methoxy-4-trifluormethoxyphenyl)-6-ethyl-3,6-dihydro-1,3,4-thiadiazin-2-on
mit Trifluoracetylchlorid:
   3-(4-Trifluoracetamidobenzyl)-5-(3-methoxy-4-trifluormethoxyphenyl)-6-ethyl-3,6-dihydro-1,3,4-thiadiazin-2-on;
mit Acetylchlorid:
   3-(4-Acetamidobenzyl)-5-(3-methoxy-4-trifluormethoxyphenyl)-6-ethyl-3,6-dihydro-1,3,4-thiadiazin-2-on;
mit Methylsulfonylchlorid:
   3-(4-Methylsulfonamidobenzyl)-5-(3-methoxy-4-trifluormethoxyphenyl)-6-ethyl-3,6-dihydro-1,3,4-thiadiazin-2-on;
mit Propionylchlorid:
   3-(4-Propionylaminobenzyl)-5-(3-methoxy-4-trifluormethoxyphenyl)-6-ethyl-3,6-dihydro-1,3,4-thiadiazin-2-on;
mit Isobutyrylchlorid:
   3-(4-lsobutyrylaminobenzyl)-5-(3-methoxy-4-trifluormethoxyphenyl)-6-ethyl-3,6-dihydro-1,3,4-thiadiazin-2-on;
mit Chlorameisensäuremethylester:
   3-(4-Methoxycarbonylaminobenzyl)-5-(3-methoxy-4-trifluormethoxyphenyl)-6-ethyl-3,6-dihydro-1,3,4-thiadiazin-2-on;
mit Chlorameisensäureethylester:
   3-(4-Ethoxycarbonylaminobenzyl)-5-(3-methoxy-4-trifluormethoxyphenyl)-6-ethyl-3,6-dihydro-1,3,4-thiadiazin-2-on;
mit Methoxalylchlorid:
   3-(4-Methoxalylaminobenzyl)-5-(3-methoxy-4-trifluormethoxyphenyl)-6-ethyl-3,6-dihydro-1,3,4-thiadiazin-2-on;
mit Chlorformamid:
   3-(4-Ureidobenzyl)-5-(3-methoxy-4-trifluormethoxyphenyl)-6-ethyl-3,6-dihydro-1,3,4-thiadiazin-2-on;
mit Butyrylchlorid:
   3-(4-Butyrylaminobenzyl)-5-(3-methoxy-4-trifluormethoxyphenyl)-6-ethyl-3,6-dihydro-1,3,4-thiadiazin-2-on;
mit Pentanoylchlorid:
   3-(4-Pentanoylaminobenzyl)-5-(3-methoxy-4-trifluormethoxyphenyl)-6-ethyl-3,6-dihydro-1,3,4-thiadiazin-2-on;
mit Hexanoylchlorid:
   3-(4-Hexanoylaminobenzyl)-5-(3-methoxy-4-trifluormethoxyphenyl)-6-ethyl-3,6-dihydro-1,3,4-thiadiazin-2-on;
mit Pentafluorpropionylchlorid:
   3-(4-Pentafluorpropionylaminobenzyl)-5-(3-methoxy-4-trifluormethoxyphenyl)-6-ethyl-3,6-dihydro-1,3,4-thiadiazin-2-on;
mit Pivalylchlorid:
   3-(4-Pivalylaminobenzyl)-5-(3-methoxy-4-trifluormethoxyphenyl)-6-ethyl-3,6-dihydro-1,3,4-thiadiazin-2-on;
mit Cyclopentancarbonsäurechlorid:
   3-(4-Cyclopentylcarbamoylbenzyl)-5-(3-methoxy-4-trifluormethoxyphenyl)-6-ethyl-3,6-dihydro-1,3,4-thiadiazin-2-on.

Analog erhält man durch Umsetzung von 3-(4-Aminobenzyl)-5-(3,4-dimethoxyphenyl)-3,6-dihydro-1,3,4-thiadiazin-2-on
mit Trifluoracetylchlorid:
   3-(4-Trifluoracetamidobenzyl)-5-(3,4-dimethoxyphenyl)-3,6-dihydro-1,3,4-thiadiazin-2-on, F. 176°;
mit Acetylchlorid:
   3-(4-Acetamidobenzyl)-5-(3,4-dimethoxyphenyl)-3,6-dihydro-1,3,4-thiadiazin-2-on, F. 186°;
mit Methylsulfonylchlorid:
   3-(4-Methylsulfonamidobenzyl)-5-(3,4-dimethoxyphenyl)-3,6-dihydro-1,3,4-thiadiazin-2-on;
mit Propionylchlorid:
   3-(4-Propionylaminobenzyl)-5-(3,4-dimethoxyphenyl)-3,6-dihydro-1,3,4-thiadiazin-2-on, F. 186°;
mit Isobutyrylchlorid:
   3-(4-lsobutyrylaminobenzyl)-5-(3,4-dimethoxyphenyl)-3,6-dihydro-1,3,4-thiadiazin-2-on, F. 137°;
mit Chlorameisensäuremethylester:
   3-(4-Methoxycarbonylaminobenzyl)-5-(3,4-dimethoxyphenyl)-3,6-dihydro-1,3,4-thiadiazin-2-on;
mit Chlorameisensäureethylester:
   3-(4-Ethoxycarbonylaminobenzyl)-5-(3,4-dimethoxyphenyl)-3,6-dihydro-1,3,4-thiadiazin-2-on;
mit Pivalylchlorid:
   3-(4-Pivalylaminobenzyl)-5-(3,4-dimethoxyphenyl)-3,6-dihydro-1,3,4-thiadiazin-2-on;
mit Methoxalylchlorid:
   3-(4-Methoxalylaminobenzyl)-5-(3,4-dimethoxyphenyl)-3,6-dihydro-1,3,4-thiadiazin-2-on;
mit Chlorformamid:
   3-(4-Ureidobenzyl)-5-(3,4-dimethoxyphenyl)-3,6-dihydro-1,3,4-thiadiazin-2-on;
mit Butyrylchlorid:
   3-(4-Butyrylaminobenzyl)-5-(3,4-dimethoxyphenyl)-3,6-dihydro-1,3,4-thiadiazin-2-on;
mit Pentanoylchlorid:
   3-(4-Pentanoylaminobenzyl)-5-(3,4-dimethoxyphenyl)-3,6-dihydro-1,3,4-thiadiazin-2-on;
mit Hexanoylchlorid:
   3-(4-Hexanoylaminobenzyl)-5-(3,4-dimethoxyphenyl)-3,6-dihydro-1,3,4-thiadiazin-2-on;
mit Pentafluorpropionylchlorid:
   3-(4-Pentafluorpropionylaminobenzyl)-5-(3,4-dimethoxyphenyl)-3,6-dihydro-1,3,4-thiadiazin-2-on;
mit Cyclopentancarbonsäurechlorid:
   3-(4-Cyclopentylcarbamoylbenzyl)-5-(3,4-dimethoxyphenyl)-3,6-dihydro-1,3,4-thiadiazin-2-on.

Analog erhält man durch Umsetzung von 3-(4-Aminobenzyl)-5-(3-ethoxy-4-methoxyphenyl)-3,6-dihydro-1,3,4-thiadiazin-2-on
mit Trifluoracetylchlorid:
   3-(4-Trifluoracetamidobenzyl)-5-(3-ethoxy-4-methoxyphenyl)-3,6-dihydro-1,3,4-thiadiazin-2-on, F. 188°;
mit Acetylchlorid:
   3-(4-Acetamidobenzyl)-5-(3-ethoxy-4-methoxyphenyl)-3,6-dihydro-1,3,4-thiadiazin-2-on;
mit Methylsulfonylchlorid:
   3-(4-Methylsulfonamidobenzyl)-5-(3-ethoxy-4-methoxyphenyl)-3,6-dihydro-1,3,4-thiadiazin-2-on;
mit Propionylchlorid:
   3-(4-Propionylaminobenzyl)-5-(3-ethoxy-4-methoxyphenyl)-3,6-dihydro-1,3,4-thiadiazin-2-on, F. 184°;
mit Isobutyrylchlorid:
   3-(4-Isobutyrylaminobenzyl)-5-(3-ethoxy-4-methoxyphenyl)-3,6-dihydro-1,3,4-thiadiazin-2-on;
mit Chlorameisensäuremethylester:
   3-(4-Methoxycarbonylaminobenzyl)-5-(3-ethoxy-4-methoxyphenyl)-3,6-dihydro-1,3,4-thiadiazin-2-on;
mit Chlorameisensäureethylester:
   3-(4-Ethoxycarbonylaminobenzyl)-5-(3-ethoxy-4-methoxyphenyl)-3,6-dihydro-1,3,4-thiadiazin-2-on, F. 95°;
mit Pivalylchlorid:
   3-(4-Pivalylaminobenzyl)-5-(3-ethoxy-4-methoxyphenyl)-3,6-dihydro-1,3,4-thiadiazin-2-on;
mit Methoxalylchlorid:
   3-(4-Methoxalylaminobenzyl)-5-(3-ethoxy-4-methoxyphenyl)-3,6-dihydro-1,3,4-thiadiazin-2-on;
mit Chlorformamid:
   3-(4-Ureidobenzyl)-5-(3-ethoxy-4-methoxyphenyl)-3,6-dihydro-1,3,4-thiadiazin-2-on;
mit Butyrylchlorid:
   3-(4-Butyrylaminobenzyl)-5-(3-ethoxy-4-methoxyphenyl)-3,6-dihydro-1,3,4-thiadiazin-2-on;
mit Pentanoylchlorid:
   3-(4-Pentanoylaminobenzyl)-5-(3-ethoxy-4-methoxyphenyl)-3,6-dihydro-1,3,4-thiadiazin-2-on;
mit Hexanoylchlorid:
   3-(4-Hexanoylaminobenzyl)-5-(3-ethoxy-4-methoxyphenyl)-3,6-dihydro-1,3,4-thiadiazin-2-on;
mit Pentafluorpropionylchlorid:
   3-(4-Pentafluorpropionylaminobenzyl)-5-(3-ethoxy-4-methoxyphenyl)-3,6-dihydro-1,3,4-thiadiazin-2-on;
mit Cyclopentancarbonsäurechlorid:
   3-(4-Cyclopentylcarbamoylbenzyl)-5-(3-ethoxy-4-methoxyphenyl)-3,6-dihydro-1,3,4-thiadiazin-2-on.

Analog erhält man durch Umsetzung von 3-(4-Aminobenzyl)-5-(3-cyclopentyloxy-4-methoxyphenyl)-3,6-dihydro-1,3,4-thiadiazin-2-on
mit Trifluoracetylchlorid:
   3-(4-Trifluoracetamidobenzyl)-5-(3-cyclopentyloxy-4-methoxyphenyl)-3,6-dihydro-1,3,4-thiadiazin-2-on, F. 196°;
mit Acetylchlorid:
   3-(4-Acetamidobenzyl)-5-(3-cyclopentyloxy-4-methoxyphenyl)-3,6-dihydro-1,3,4-thiadiazin-2-on;
mit Methylsulfonylchlorid:
   3-(4-Methylsulfonamidobenzyl)-5-(3-cyclopentyloxy-4-methoxyphenyl)-3,6-dihydro-1,3,4-thiadiazin-2-on;
mit Propionylchlorid:
   3-(4-Propionylaminobenzyl)-5-(3-cyclopentyloxy-4-methoxyphenyl)-3,6-dihydro-1,3,4-thiadiazin-2-on, F. 103°;
mit Isobutyrylchlorid:
   3-(4-lsobutyrylaminobenzyl)-5-(3-cyclopentyloxy-4-methoxyphenyl)-3,6-dihydro-1,3,4-thiadiazin-2-on;
mit Chlorameisensäuremethylester:
   3-(4-Methoxycarbonylaminobenzyl)-5-(3-cyclopentyloxy-4-methoxyphenyl)-3,6-dihydro-1,3,4-thiadiazin-2-on;
mit Chlorameisensäureethylester:
   3-(4-Ethoxycarbonylaminobenzyl)-5-(3-cyclopentyloxy-4-methoxyphenyl)-3,6-dihydro-1,3,4-thiadiazin-2-on, F. 72°;
mit Pivalylchlorid:
   3-(4-Pivalylaminobenzyl)-5-(3-cyclopentyloxy-4-methoxyphenyl)-3,6-dihydro-1,3,4-thiadiazin-2-on;
mit Methoxalylchlorid:
   3-(4-Methoxalylaminobenzyl)-5-(3-cyclopentyloxy-4-methoxyphenyl)-3,6-dihydro-1,3,4-thiadiazin-2-on;
mit Chlorformamid:
   3-(4-Ureidobenzyl)-5-(3-cyclopentyloxy-4-methoxyphenyl)-3,6-dihydro-1,3,4-thiadiazin-2-on;
mit Butyrylchlorid:
   3-(4-Butyrylaminobenzyl)-5-(3-cyclopentyloxy-4-methoxyphenyl)-3,6-dihydro-1,3,4-thiadiazin-2-on;
mit Pentanoylchlorid:
   3-(4-Pentanoylam inobenzyl)-5-(3-cyclopentyloxy-4-methoxyphenyl)-3,6-dihydro-1,3,4-thiadiazin-2-on;
mit Hexanoylchlorid:
   3-(4-Hexanoylaminobenzyl)-5-(3-cyclopentyloxy-4-methoxyphenyl)-3,6-dihydro-1,3,4-thiadiazin-2-on;
mit Pentafluorpropionylchlorid:
   3-(4-Pentafluorpropionylaminobenzyl)-5-(3-cyclopentyloxy-4-methoxyphenyl)-3,6-dihydro-1,3,4-thiadiazin-2-on;
mit Cyclopentancarbonsäurechlorid:
   3-(4-Cyclopentylcarbamoylbenzyl)-5-(3-cyclopentyloxy-4-methoxyphenyl)-3,6-dihydro-1,3,4-thiadiazin-2-on.

Analog erhält man durch Umsetzung von 3-(4-Aminophenethyl)-5-(3,4-dimethoxyphenyl)-3,6-dihydro-1,3,4-thiadiazin-2-on
mit Trifluoracetylchlorid:
   3-(4-Trifluoracetamidophenethyl)-5-(3,4-dimethoxyphenyl)-3,6-dihydro-1,3,4-thiadiazin-2-on;
mit Acetylchlorid:
   3-(4-Acetamidophenethyl)-5-(3,4-dimethoxyphenyl)-3,6-dihydro-1,3,4-thiadiazin-2-on;
mit Methylsulfonylchlorid:
   3-(4-Methylsulfonamidophenethyl)-5-(3,4-dimethoxyphenyl)-3,6-dihydro-1,3,4-thiadiazin-2-on;
mit Propionylchlorid:
   3-(4-Propionylaminophenethyl)-5-(3,4-dimethoxyphenyl)-3,6-dihydro-1,3,4-thiadiazin-2-on;
mit Isobutyrylchlorid:
   3-(4-lsobutyrylaminophenethyl)-5-(3,4-dimethoxyphenyl)-3,6-dihydro-1,3,4-thiadiazin-2-on;
mit Chlorameisensäuremethylester:
   3-(4-Methoxycarbonylaminophenethyl)-5-(3,4-dimethoxyphenyl)-3,6-dihydro-1,3,4-thiadiazin-2-on;
mit Chlorameisensäureethylester:
   3-(4-Ethoxycarbonylaminophenethyl)-5-(3,4-dimethoxyphenyl)-3,6-dihydro-1,3,4-thiadiazin-2-on;
mit Pivalylchlorid:
   3-(4-Pivalylaminophenethyl)-5-(3,4-dimethoxyphenyl)-3,6-dihydro-1,3,4-thiadiazin-2-on;
mit Methoxalylchlorid:
   3-(4-Methoxalylaminophenethyl)-5-(3,4-dimethoxyphenyl)-3,6-dihydro-1,3,4-thiadiazin-2-on;
mit Chlorformamid:
   3-(4-Ureidophenethyl)-5-(3,4-dimethoxyphenyl)-3,6-dihydro-1,3,4-thiadiazin-2-on;
mit Butyrylchlorid:
   3-(4-Butyrylaminophenethyl)-5-(3,4-dimethoxyphenyl)-3,6-dihydro-1,3,4-thiadiazin-2-on;
mit Pentanoylchlorid:
   3-(4-Pentanoylaminophenethyl)-5-(3,4-dimethoxyphenyl)-3,6-dihydro-1,3,4-thiadiazin-2-on;
mit Hexanoylchlorid:
   3-(4-Hexanoylaminophenethyl)-5-(3,4-dimethoxyphenyl)-3,6-dihydro-1,3,4-thiadiazin-2-on;
mit Pentafluorpropionylchlorid:
   3-(4-Pentafluorpropionylaminophenethyl)-5-(3,4-dimethoxyphenyl)-3,6-dihydro-1,3,4-thiadiazin-2-on;
mit Cyclopentancarbonsäurechlorid:
   3-(4-Cyclopentylcarbamoylphenethyl)-5-(3,4-dimethoxyphenyl)-3,6-dihydro-1,3,4-thiadiazin-2-on.

Analog erhält man durch Umsetzung von 3-(3-Aminobenzyl)-5-(3,4-dimethoxyphenyl)-3,6-dihydro-1,3,4-thiadiazin-2-on
mit Trifluoracetylchlorid:
   3-(3-Trifluoracetamidobenzyl)-5-(3,4-dimethoxyphenyl)-3,6-dihydro-1,3,4-thiadiazin-2-on;
mit Acetylchlorid:
   3-(3-Acetamidobenzyl)-5-(3,4-dimethoxyphenyl)-3,6-dihydro-1,3,4-thiadiazin-2-on;
mit Methylsulfonylchlorid:
   3-(4-Methylsulfonamidobenzyl)-5-(3,4-dimethoxyphenyl)-3,6-dihydro-1,3,4-thiadiazin-2-on;
mit Propionylchlorid:
   3-(3-Propionylaminobenzyl)-5-(3,4-dimethoxyphenyl)-3,6-dihydro-1,3,4-thiadiazin-2-on;
mit Isobutyrylchlorid:
   3-(3-lsobutyrylaminobenzyl)-5-(3,4-dimethoxyphenyl)-3,6-dihydro-1,3,4-thiadiazin-2-on;
mit Chlorameisensäuremethylester:
   3-(3-Methoxycarbonylaminobenzyl)-5-(3,4-dimethoxyphenyl)-3,6-dihydro-1,3,4-thiadiazin-2-on;
mit Chlorameisensäureethylester:
   3-(3-Ethoxycarbonylaminobenzyl)-5-(3,4-dimethoxyphenyl)-3,6-dihydro-1,3,4-thiadiazin-2-on;
mit Pivalylchlorid:
   3-(3-Pivalylaminobenzyl)-5-(3,4-dimethoxyphenyl)-3,6-dihydro-1,3,4-thiadiazin-2-on;
mit Methoxalylchlorid:
   3-(3-Methoxalylaminobenzyl)-5-(3,4-dimethoxyphenyl)-3,6-dihydro-1,3,4-thiadiazin-2-on;
mit Chlorformamid:
   3-(3-Ureidobenzyl)-5-(3,4-dimethoxyphenyl)-3,6-dihydro-1,3,4-thiadiazin-2-on;
mit Butyrylchlorid:
   3-(3-Butyrylaminobenzyl)-5-(3,4-dimethoxyphenyl)-3,6-dihydro-1,3,4-thiadiazin-2-on;
mit Pentanoylchlorid:
   3-(3-Pentanoylam inobenzyl)-5-(3,4-dimethoxyphenyl)-3,6-dihydro-1,3,4-thiadiazin-2-on;
mit Hexanoylchlorid:
   3-(3-Hexanoylaminobenzyl)-5-(3,4-dimethoxyphenyl)-3,6-dihydro-1,3,4-thiadiazin-2-on;
mit Pentafluorpropionylchlorid:
   3-(3-Pentafluorpropionylam inobenzyl)-5-(3,4-dimethoxyphenyl)-3,6-dihydro-1,3,4-thiadiazin-2-on;
mit Cyclopentancarbonsäurechlorid:
   3-(3-Cyclopentylcarbamoylbenzyl)-5-(3,4-dimethoxyphenyl)-3,6-dihydro-1,3,4-thiadiazin-2-on.

### Beispiel 7

Eine Lösung 1,4 g 3-(4-Aminobenzyl)-5-(3,4-dimethoxyphenyl)-6-ethyl-3,6-dihydro-1,3,4-thiadiazin-2-on in 60 ml Dichlormethan und 1 ml Triethylamin wird unter Kühlung und Rühren mit 1,5 ml Butylbromid, gelöst in 20 ml Dichlormethan, versetzt und 3 Stunden nachgerührt. Man entfernt das Lösungsmittel und arbeitet wie üblich auf. Man erhält 3-(4-N,N-Dibutylaminobenzyl)-5-(3,4-dimethoxy-phenyl)-6-ethyl-3,6-dihydro-1,3,4-thiadiazin-2-on.

### Beispiel 8

Eine Lösung von 1,4 g 3-(4-Propionyl-aminobenzyl)-5-(3-hydroxy-4-methoxyphenyl)-6-ethyl-3,6-dihydro-1,3,4-thiadiazin-2-on [erhältlich durch Umsetzung von 3-(4-Aminobenzyl)-5-(3-hydroxy-4-methoxyphenyl)-6-ethyl-3,6-dihydro-1,3,4-thiadiazin-2-on mit Propionylchlorid] in THF wird nach Zugabe von einem Äquivalent Trifluormethyljodid zwei Stunden gekocht.

Anschließend wird das Lösungsmittel entfernt und wie üblich aufgearbeitet. Man erhält 3-(4-Propionylamino)-5-(3-trifluormethoxy-4-methoxyphenyl)-6-ethyl-3,6-dihydro-1,3,4-thiadiazin-2-on.

### Beispiel 9

Analog Beispiel 4 erhält man durch Umsetzung
von 3-(2-Nitrobenzyl)-5-(3,4-dimethoxy)-3,6-dihydro-1,3,4-thiadiazin-2-on:
   3-(2-Aminobenzyl)-5-(3,4-dimethoxy)-3,6-dihydro-1,3,4-thiadiazin-2-on ("E"), F. 127°;
von 3-(4-Nitrobenzyl)-5-(3-propoxy-4-methoxy)-3,6-dihydro-1,3,4-thiadiazin-2-on:
   3-(4-Aminobenzyl)-5-(3-propoxy-4-methoxy)-3,6-dihydro-1,3,4-thiadiazin-2-on, F. 125°;
von 3-(4-Nitrobenzyl)-5-(3-cyclopentyloxy-4-methoxy)-3,6-dihydro-1,3,4-thiadiazin-2-on:
   3-(4-Aminobenzyl)-5-(3-cyclopentyloxy-4-methoxy)-3,6-dihydro-1,3,4-thiadiazin-2-on, F. 123°.

### Beispiel 10

Analog Beispiel 6 erhält man durch Umsetzung von "E"
mit Acetylchlorid:
   3-(2-Acetamidobenzyl)-5-(3,4-dimethoxy)-3,6-dihydro-1,3,4-thiadiazin-2-on, F. 210°;
mit Trifluoracetylchlorid:
   3-(2-Trifluoracetamidobenzyl)-5-(3,4-dimethoxy)-3,6-dihydro-1,3,4-thiadiazin-2-on, F. 200°.

Analog erhält man durch Umsetzung von 3-(4-Aminobenzyl)-5-(3-propoxy-4-methoxy)-3,6-dihydro-1,3,4-thiadiazin-2-on
mit Acetylchlorid:
   3-(4-Acetamidobenzyl)-5-(3-propoxy-4-methoxy))-3,6-dihydro-1,3,4-thiadiazin-2-on, kein definierter Schmelzpunkt, amorph.;
mit Propionylchlorid:
   3-(4-Propionylaminobenzyl)-5-(3-propoxy-4-methoxy))-3,6-dihydro-1, 3,4-thiadiazin-2-on, F. 150°;
mit Trifluoracetylchlorid:
   3-(4-Trifluoracetamido)-5-(3-propoxy-4-methoxy))-3,6-dihydro-1,3,4-thiadiazin-2-on, F. 167°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ 2 H₂O, 28,48 g Na₂HPO₄. 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Arylalkyl-thiadiazinonderivate der Formel I 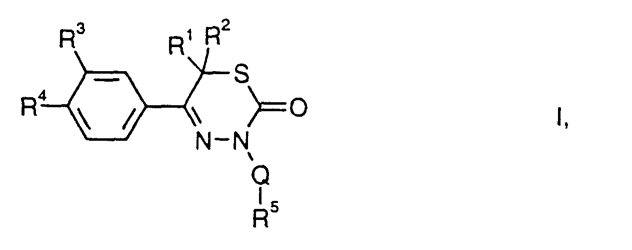 worin
R¹ und R² jeweils unabängig voneinander H oder A,
R³ und R⁴ jeweils unabhängig voneinander -OH, -OR¹⁰, -S-R¹⁰, -SO-R¹⁰, -SO₂-R¹⁰, Hal, Methylendioxy, -NO₂, -NH₂,-NHR¹⁰ oder -NR¹⁰R¹¹,
R⁵ einen unsubstituierten oder ein- oder zweifach durch R⁶ und/oder R⁷ substituierten Phenylrest,
Q Alkylen mit 1-6 C-Atomen,
R⁶ und R⁷ jeweils unabhängig voneinander -NH₂, -NR⁸R⁹,-NHR¹⁰, -NR¹⁰R¹¹, -NO₂, Hal, -CN, -OA, -COOH oder-COOA,
R⁸ und R⁹ jeweils unabhängig voneinander H, Acyl mit 1-8 C-Atomen, das durch 1-5 F- und/oder Cl-Atome substituiert sein kann, -COOA, -S-A, -SO-A, -SO₂A,-CONH₂, -CONHA, -CONA₂, -CO-COOH, -CO-COOA, -CO-CONH₂, -CO-CONHA oder -CO-CONA₂,
A Alkyl mit 1-6 C-Atomen, das durch 1-5 F- und/oder Cl-Atome substituiert sein kann,
R¹⁰ und R¹¹ jeweils unabhängig voneinander A, Cycloalkyl mit 3-7 C-Atomen, Methylencycloalkyl mit 4-8 C-Atomen oder Alkenyl mit 2-8 C-Atomen
und
Hal F, Cl, Br oder I
bedeuten,
sowie deren physiologisch unbedenklichen Salze.

2. Ein Enantiomer einer Verbindung der Formel I gemäß Anspruch 1.

3. (a) 3-(4-Nitrobenzyl)-5-(3,4-dimethoxyphenyl)-6-ethyl-3,6-dihydro-1,3,4-thiadiazin-2-on;
(b) 3-(4-Aminobenzyl)-5-(3,4-dimethoxyphenyl)-6-ethyl-3,6-dihydro-1,3,4-thiadiazin-2-on;
(c) 3-(4-Trifluoracetamidobenzyl)-5-(3,4-dimethoxyphenyl)-6-ethyl-3,6-dihydro-1,3,4-thiadiazin-2-on;
(d) 3-(4-Acetamidobenzyl)-5-(3,4-dimethoxy-phenyl)-3,6-dihydro-1,3,4-thiadiazin-2-on;
(e) 3-(4-Methoxybenzyl)-5-(3,4-dimethoxy-phenyl)-6-ethyl-3,6-dihydro-1,3,4-thiadiazin-2-on;
(f) 3-(2,6-Dichlorbenzyl)-5-(3,4-dimethoxy-phenyl)-6-ethyl-3,6-dihydro-1,3,4-thiadiazin-2-on.

4. Verfahren zur Herstellung von Verbindungen der Formel I nach Anspruch 1 sowie deren Salzen, dadurch gekennzeichnet, daß man eine Verbindung der Formel II 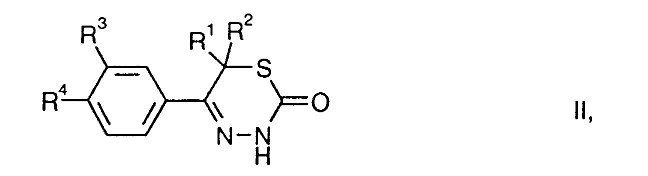 worin
R¹, R², R³ und R⁴ die angegebenen Bedeutungen haben,
mit einer Verbindung der Formel III
R⁵-Q-X III,
worin
R⁵ und Q die angegebenen Bedeutungen haben, und
X Cl, Br, OH oder eine reaktionsfähige veresterte OH-Gruppe bedeutet,
umsetzt,
oder daß man in einer Verbindung der Formel I einen Rest R⁵ in einen anderen Rest R⁵ umwandelt, indem man eine Nitrogruppe reduziert, eine primäre oder eine sekundäre Aminogruppe alkyliert oder acyliert oder eine Cyangruppe hydrolysiert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ und X die angegebenen Bedeutungen haben, umsetzt und/oder eine Base der Formel I durch Behandeln mit einer Säure in eines ihrer Salze umwandelt.

5. Verfahren zur Herstellung pharmazeutischer Zubereitungen, dadurch gekennzeichnet, daß man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

6. Pharmazeutische Zubereitung, gekennzeichnet durch einen Gehalt an mindestens einer Verbindung der Formel I nach Anspruch 1 und/oder einem ihrer physiologisch unbedenklichen Salze.

7. Verwendung einer Verbindung der Formel I nach Anspruch 1 und/ oder eines ihrer physiologisch unbedenklichen Salze zur Herstellung eines Arzneimittels zur Bekämpfung von Krankheiten.

## Claims

1. Arylalkyl-thiadiazinone derivatives of the formula I 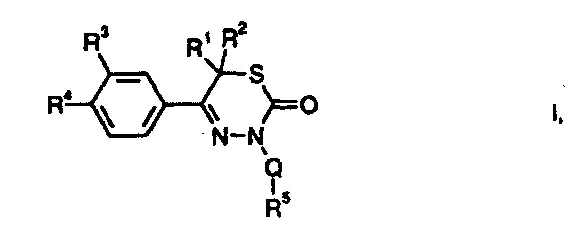 in which
R¹ and R² each independently of one another are H or A,
R³ and R⁴ each independently of one another are -OH, -OR¹⁰, -S-R¹⁰, -SO-R¹⁰, -SO₂-R¹⁰, Hal, methylenedioxy, -NO₂, -NH₂, -NHR¹⁰ or -NR¹⁰R¹¹,
R⁵ is a phenyl radical which is unsubstituted or is mono- or disubstituted by R⁶ and/or R⁷,
Q is alkylene having 1-6 carbon atoms,
R⁶ and R⁷ each independently of one another are -NH₂, -NR⁸R⁹, -NHR¹⁰, -NR¹⁰R¹¹, -NO₂, Hal, -CN, -OA, -COOH or -COOA,
R⁸ and R⁹ each independently of one another are H, acyl having 1-8 carbon atoms and which can be substituted by 1-5 fluorine and/or chlorine atoms, -COOA, -S-A, -SO-A, -SO₂A, -CONH₂, -CONHA, -CONA₂, -CO-COOH, -CO-COOA, -CO-CONH₂, -CO-CONHA or -CO-CONA₂,
A is alkyl having 1-6 carbon atoms and which can be substituted by 1-5 fluorine and/or chlorine atoms,
R¹⁰ and R¹¹ each independently of one another are A, cycloalkyl having 3-7 carbon atoms, methylenecycloalkyl having 4-8 carbon atoms or alkenyl having 2-8 carbon atoms
and
Hal is F, Cl, Br or I,
and physiologically unobjectionable salts thereof.

2. An enantiomer of a compound of the formula I according to Claim 1.

3. a) 3-(4-Nitrobenzyl)-5-(3,4-dimethoxyphenyl)-6-ethyl-3,6-dihydro-1,3,4-thiadiazin-2-one;
(b) 3-(4-aminobenzyl)-5-(3,4-dimethoxyphenyl)-6-ethyl-3,6-dihydro-1,3,4-thiadiazin-2-one;
(c) 3-(4-trifluoroacetamidobenzyl)-5-(3,4-dimethoxyphenyl)-6-ethyl-3,6-dihydro-1,3,4-thiadiazin-2-one;
(d) 3-(4-acetamidobenzyl)-5-(3,4-dimethoxyphenyl)-3,6-dihydro-1,3,4-thiadiazin-2-one;
(e) 3- (4-methoxybenzyl) -5-(3,4-dimethoxyphenyl)-6-ethyl-3,6-dihydro-1,3,4-thiadiazin-2-one;
(f) 3-(2,6-dichlorobenzyl)-5-(3,4-dimethoxyphenyl) -6-ethyl-3,6-dihydro-1,3,4-thiadiazin-2-one.

4. Process for the preparation of compounds of the formula I according to Claim 1 and of salts thereof, characterized in that a compound of the formula II 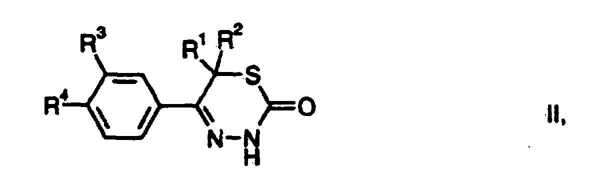 in which
R¹, R², R³ and R⁴ have the given meanings, is reacted with a compound of the formula III
**R**^{**5**}**-Q-X** **III,**
in which
R⁵ and Q have the meanings given and
x is Cl, Br, OH or a reactive esterified OH group,
or in that, in a compound of the formula I, one radical R⁵ is converted into another radical R⁵ by reducing a nitro group, acylating or alkylating a primary or a secondary amino group, or hydrolysing a cyano group, and/or in that a compound corresponding to the formula I but containing one or two free OH groups instead of R³ and/or R⁴ is if appropriate reacted with a compound of the formula R³-X or R⁴-X, respectively, in which R³, R⁴ and X have the meanings given, and/or a base of the formula I is converted into one of its salts by treatment with an acid.

5. Process for the production of pharmaceutical preparations, characterized in that a compound of the formula I according to Claim 1 and/or one of its physiologically unobjectionable salts is brought, together with at least one solid, liquid or semi-liquid excipient or auxiliary, into a suitable dosage form.

6. Pharmaceutical preparation, characterized by a content of at least one compound of the formula I according to Claim 1 and/or one of its physiologically unobjectionable salts.

7. Use of a compound of the formula I according to Claim 1 and/or one of its physiologically unobjectionable salts for the preparation of a medicament for combating diseases.

## Revendications

1. Les dérivés des arylalkylthiadiazinones de formule I 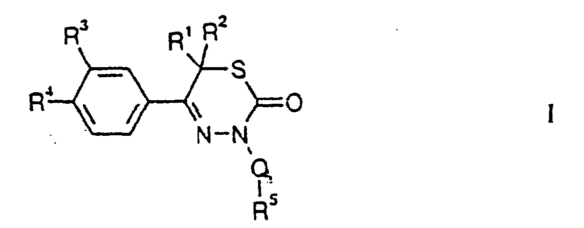 où
R¹ et R² représentent, indépendamment l'un de l'autre, H ou A,
R³ et R⁴ représentent, indépendamment l'un de l'autre, -OH, -OR¹⁰, -S-R¹⁰, -SO-R¹⁰, -SO₂-R¹⁰, Hal, le méthylènedioxy, -NO₂, - NH₂, -NHR¹⁰ ou -NR¹⁰R¹¹,
R⁵ représente un reste phényle non substitué ou mono- ou disubstitué par R⁶ et/ou R⁷,
Q un alkylène comportant 1 à 6 atomes de C,
R⁶ et R⁷ représentent, indépendamment l'un de l'autre, -NH₂, -NR⁸R⁹, -NHR¹⁰, -NR¹⁰R¹¹, -NO₂, Hal, -CN, -OA, -COOH ou -COOA,
R⁸ et R⁹ représentent, indépendamment l'un de l'autre, H, un acyle pouvant être substitué par 1 à 5 atomes de F et/ou de Cl et comportant 1 à 8 atomes de C, -COOA, -S-A, -SO-A, -SO₂A, -CONH₂, -CONHA, -CONA₂, -CO-COOH, -CO-COOA, -CO-CONH₂, -CO-CONHA ou -CO-CONA₂,
A représente un alkyle comportant 1 à 6 atomes de C, pouvant être substitué par 1 à 5 atomes de F et/ou de Cl,
R¹⁰ et R¹¹ représentent, indépendamment l'un de l'autre, A, un cycloalkyle comportant 3 à 7 atomes de C, un méthylènecycloalkyle comportant 4 à 8 atomes de C ou un alcényle comportant 2 à 8 atomes de C, et
Hal représente F, Cl, Br ou I,
ainsi que leurs sels physiologiquement acceptables.

2. Un énantiomère d'un composé de formule I selon la revendication 1.

3. (a) la 3-(4-nitrobenzyl)-5-(3,4-diméthoxyphényl)-6-éthyl-3,6-dihydro-1,3,4-thiadiazin-2-one,
(b) la 3-(4-aminobenzyl)-5-(3,4-diméthoxyphényl)-6-éthyl-3,6-dihydro-1,3,4-thiadiazin-2-one,
(c) la 3-(4-trifluoroacétamidobenzyl)-5-(3,4-diméthoxyphényl)-6-éthyl-3,6-dihydro-1,3,4-thiadiazin-2-one,
(d) la 3-(4-acétamidobenzyl)-5-(3,4-diméthoxyphényl)-3,6-dihydro-1, 3,4-thiadiazin-2-one,
(e) la 3-(4-méthoxybenzyl)-5-(3,4-diméthoxyphényl)-6-éthyl-3,6-dihydro-1,3,4-thiadiazin-2-one,
(f) la 3-(2,6-dichlorobenzyl)-5-(3,4-diméthoxyphényl)-6-éthyl-3,6-dihydro-1,3,4-thiadiazin-2-one.

4. Procédé pour la préparation des composés de formule I selon la revendication 1, ainsi que de leurs sels, caractérisé en ce que l'on fait réagir un composé de formule II 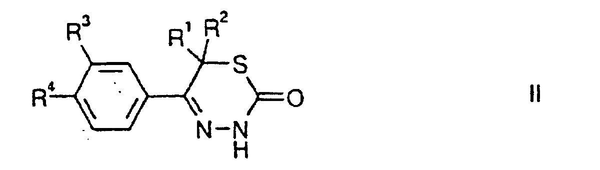 où
R¹, R², R³ et R⁴ ont les significations indiquées,
avec un composé de formule III
R⁵-Q-X III
où
R⁵ et Q ont les significations indiquées et
X représente Cl, Br, OH ou un groupe OH estérifié réactif,
ou en ce que l'on transforme dans un composé de formule I, un reste R⁵ en un autre reste R⁵, en réduisant un groupe nitro, en alcoylant ou en acylant un groupe amino primaire ou secondaire ou en hydrolysant un groupe cyano,
et/ou en ce que l'on fait éventuellement réagir un composé correspondant à la formule I, mais contenant à la place de R³ et/ou de R⁴ un ou deux groupes OH libres, avec un composé de formule R³-X ou R⁴-X où R³, R⁴ et X ont les significations indiquées et/ou en ce que l'on transforme une base de formule I en l'un de ses sels par traitement avec un acide.

5. Procédé pour la fabrication de préparations pharmaceutiques, caractérisé en ce que l'on met sous une forme de dosage appropriée un composé de formule I selon la revendication 1 et/ou l'un de ses sels physiologiquement acceptables, associé à au moins un support ou un adjuvant solide, liquide ou semi-liquide.

6. Préparation pharmaceutique, caractérisée en ce qu'elle contient au moins un composé de formule I selon la revendication 1 et/ou l'un de ses sels physiologiquement acceptables.

7. Utilisation d'un composé de formule I selon la revendication 1 et/ou de l'un de ses sels physiologiquement acceptables pour la préparation d'un médicament destiné à lutter contre les maladies.
